# EUROPÄISCHE PATENTSCHRIFT

(11) **EP 4 016 543 B1**
(45) Veröffentlichungstag und Bekanntmachung des Hinweises auf die Patenterteilung: **13.08.2025**
(21) Anmeldenummer: 21207429.8
(22) Anmeldetag: 10.11.2021
(51) Int. Cl.: G06N 3/045, G06N 3/084, G06T 7/00, G16H 30/40, G16H 50/20, G06N 20/00

(54) **VERFAHREN UND VORRICHTUNG ZUR BEREITSTELLUNG EINER MEDIZINISCHEN INFORMATION**
METHOD AND DEVICE FOR THE PROVISION OF MEDICAL INFORMATION
PROCÉDÉ ET DISPOSITIF DE FOURNITURE DES INFORMATIONS MÉDICALES

(30) Priorität: 15.12.2020 DE 102020215943
(43) Veröffentlichungstag der Anmeldung: 22.06.2022
(73) Patentinhaber: Siemens Healthineers AG, 91301 Forchheim (DE)
(72) Erfinder: Huber, Martin, 91080 Uttenreuth (DE); Kohle, Sven, 91056 Erlangen (DE); Speier, Christoph, 91052 Erlangen (DE)
(74) Vertreter: Siemens Healthineers Patent Attorneys

(56) Entgegenhaltungen:
- US-A1- 2012 114 256
- US-A1- 2016 267 221
- US-A1- 2019 117 978
- US-A1- 2020 380 675

## Beschreibung

Die Erfindung betrifft Verfahren und Vorrichtungen zur Bereitstellung einer medizinischen Information. Die medizinische Information kann dabei zum Beispiel eine Angabe zu möglichen klinischen Befunden oder andere klinisch relevante Angaben für einen Patienten umfassen. Die medizinische Information kann dabei basierend auf einer (teil-)automatisierten Analyse der einem Nutzer zur Verfügung stehenden medizinischen Datensätze bereitgestellt werden.

Die Behandlung eines Patienten ist mit zahlreichen medizinischen Entscheidungen verbunden, die das Wohlbefinden des Patienten direkt beeinflussen. Die zu treffenden Entscheidungen sind vielschichtig. Sie umfassen unter anderem Therapieentscheidungen oder die Erstellung von Prognosen. Je nach Fall muss zusätzlich über Folgeuntersuchungen oder Überweisungen entschieden werden. Für alle diese Entscheidungen ist eine möglichst akkurate Diagnose des Patienten basierend auf den zur Verfügung stehenden Informationen unerlässlich.

Im modernen Krankenhausumfeld hat das medizinische Personal nahezu unbeschränkten Zugang zu allen Arten von Information zu einem vorliegenden Fall. Da medizinische Informationssysteme immer verbreiteter und leistungsfähiger werden, können potentiell entscheidungsrelevante Daten im Prinzip jederzeit abgerufen werden. Dies umfasst beispielsweise den Zugriff auf medizinische Richtlinien, auf Datenbanken mit ähnlich gelagerten Fällen oder die elektronische Patientenakte mit allen Voruntersuchungen und Vorbefunden. Dies hat den Nachteil, dass das Informationsvolumen mittlerweile so umfangreich ist, dass es von dem medizinischen Personal kaum mehr zu überblicken ist. Hier setzten so genannte "klinische Entscheidungs-Unterstützungssysteme" (ein englischer Fachbegriff hierfür ist "clinical decision support system") an. Basierend auf einer strukturierten Datenspeicherung und einer konzertierten Datenabfrage, sind solche Systeme dazu ausgelegt, relevante Informationen herauszufiltern, zu bewerten und dem Nutzer beispielsweise in Form von Vorschlägen zur Verfügung zu stellen.

Die US 2020 / 0 380 675 A1 offenbart eine automatisierte Pipeline zur akkuraten Erfassung und Segmentierung von Läsionen. Die US 2016 / 0 267 221 A1 offenbart Verfahren umfassend ein Auffinden von medizinischen Referenzbildern zu einem medizinischen Bild eines Patienten zur Befundungsunterstützung. Die US 2012/ 0 114 256 A1 offenbart ein System zum Auffinden von Vergleichsfällen basierend auf einer Ähnlichkeitsanalyse medizinischer Bilddaten. Die US 2019 / 0 117 978 A1 offenbart ein System zum Auffinden klinischer Information basieren auf einer Ähnlichkeitsanalyse von Elektrokardiogrammen.

Ein Problem mit bestehenden Systemen ist, dass sie natürlich nur mit solchen Daten arbeiten können, zu denen sie Zugang haben. Falls entscheidungsrelevante Daten fehlen, können die dem Nutzer bereitgestellten Informationen oder Vorschläge unvollständig oder schlimmstenfalls fehlerhaft sein. Dies kann insbesondere dann der Fall sein, wenn basierend auf den zur Verfügung stehenden Daten viele verschiedene Aussagen gleichermaßen in Frage kommen. Am Beispiel des Ableitens einer oder mehrerer in Frage kommender medizinischer Diagnosen kann dies in einer vergleichsweise großen Anzahl an Möglichkeiten resultieren, die einzeln betrachtet keine ausgeprägte Relevanz für den vorliegenden Fall haben. Ein sinnvolles "ranken" der in Frage kommenden medizinischen Diagnosen ist dann oft nicht möglich, da geringe Unterschiede in den Relevanzen den Ausschlag zugunsten einer möglichen Diagnose geben, die objektiv betrachtet keine maßgeblich höhere Zutreffenswahrscheinlichkeit als andere in Frage kommende Diagnosen aufweist.

Vor diesem Hintergrund ist es ein Ziel der Erfindung, verbesserte Verfahren und zugehörige Vorrichtungen bereitzustellen, die in der Lage sind, aus den zur Verfügung stehenden medizinischen Daten zu einem Patienten automatisch eine medizinische Information zu dem Patienten abzuleiten und einem Nutzer bereitzustellen. Insbesondere sollen die Verfahren und Vorrichtungen eine medizinische Information mit höherer Konfidenz und weniger Unsicherheit bereitstellen können.

Gemäß der Erfindung wird die gestellte Aufgabe mit einem Verfahren, einer Vorrichtung, einem Computerprogrammprodukt bzw. einem computerlesbaren Speichermedium gemäß dem Hauptanspruch und den nebengeordneten Ansprüchen gelöst. Vorteilhafte Weiterbildungen sind in den abhängigen Ansprüchen angegeben. Nachstehend wird die erfindungsgemäße Lösung der Aufgabe sowohl in Bezug auf die beanspruchten Vorrichtungen als auch in Bezug auf die beanspruchten Verfahren beschrieben. Hierbei erwähnte Merkmale, Vorteile oder alternative Ausführungsformen/Aspekte sind ebenso auch auf die anderen beanspruchten Gegenstände zu übertragen und umgekehrt. Mit anderen Worten können die gegenständlichen Ansprüche (die beispielsweise auf eine Vorrichtung gerichtet sind) auch mit den Merkmalen, die in Zusammenhang mit einem Verfahren beschrieben oder beansprucht sind, weitergebildet sein. Die entsprechenden funktionalen Merkmale des Verfahrens werden dabei durch entsprechende gegenständliche Module ausgebildet.

Weiterhin wird die erfindungsgemäße Lösung der Aufgabe auch in Bezug auf Verfahren und Vorrichtungen zum Anpassen von trainierten Funktionen beschrieben. Hierbei können Merkmale und alternative Ausführungsformen/Aspekte von Datenstrukturen und/oder Funktionen bei Verfahren und Vorrichtungen zur Bestimmung auf analoge Datenstrukturen und/oder Funktionen bei Verfahren und Vorrichtungen zum Anpassen übertragen werden. Analoge Datenstrukturen können hierbei insbesondere durch die Verwendung der Vorsilbe "Trainings" gekennzeichnet sein. Weiterhin können die in Verfahren und Vorrichtungen zur Bereitstellung der medizinischen Information verwendeten trainierten Funktionen insbesondere durch Verfahren und Vorrichtungen zum Anpassen von trainierten Funktionen angepasst worden und/oder bereitgestellt worden sein.

Gemäß einem Aspekt wird ein computerimplementiertes Verfahren zur Bereitstellung einer medizinischen Information bereitgestellt. Das Verfahren weist mehrere Schritte auf. Ein erster Schritt ist auf ein Empfangen eines medizinischen Datensatzes eines Patienten gerichtet. Ein weiterer Schritt ist auf ein Ermitteln eines oder mehrerer Referenzdatensätze aus mehreren Vergleichsdatensätze basierend auf Ähnlichkeitsmaßen gerichtet. Dabei basiert ein Ähnlichkeitsmaß auf der Ähnlichkeit des Datensatzes und eines der Vergleichsdatensäte. Zusätzlich ist jeder Vergleichsdatensatz mit wenigstens einem vorbekannten medizinischen Befund assoziiert. Ein weiterer Schritt ist auf ein Identifizieren eines oder mehrerer medizinischer Attribute gerichtet, welche medizinischen Attribute jeweils ein Kennzeichen eines oder mehrerer der mit den Referenzdatensätzen assoziierten vorbekannten medizinischen Befunde umfassen. Ein weiterer Schritt ist auf ein Anzeigen der identifizierten medizinischen Attribute über eine Nutzerschnittstelle gerichtet. Ein weiterer Schritt ist auf ein Empfangen einer Nutzereingabe eines Nutzers betreffend die angezeigten medizinischen Attribute über die Nutzerschnittstelle gerichtet. Ein weiterer Schritt ist auf ein Erzeugen einer medizinischen Information für den Patienten basierend auf den mit den Referenzdatensätzen assoziierten medizinischen Befunden sowie der Nutzereingabe gerichtet. Ein weiterer Schritt ist auf ein Bereitstellen der medizinischen Information gerichtet.

Dabei weist die Nutzereingabe eine Verifikation wenigstens eines Teils der identifizierten medizinischen Attribute umfassend ein Bestätigen und/oder Verwerfen eines oder mehrerer der identifizierten Attribute auf.

Der medizinische Datensatz kann dabei von einem Nutzer bereitgestellt oder ausgewählt werden. Der Nutzer kann dabei beispielsweise eine Ärztin oder ein Arzt, wie beispielsweise eine Radiologin oder ein Radiologe sein, der eine medizinische Diagnose für den Patienten erstellen will (der Patient wird nachfolgend auch "zu befundender Patient" genannt). Ferner kann der Nutzer auch der Patient selbst sein. Empfangen werden kann der medizinische Datensatz z.B. von dem Nutzer, indem er den medizinischen Datensatz z.B. hochlädt. Alternativ kann der medizinische Datensatz von einer oder mehreren entsprechenden Datenbanken empfangen werden, in denen der vollständige oder Teile des medizinischen Datensatzes gespeichert sind. Die Datenbanken können dabei beispielsweise Teil medizinischer Informationssysteme, wie etwa Krankenhausinformationssysteme (ein englischer Fachbegriff hierfür ist hospital information system oder kurz HIS) und/der PACS-Systeme (PACS steht dabei für picture archiving and communication system) und/oder Labor-Informationssysteme (LIS) sein.

Der medizinische Datensatz enthält zu dem Patienten verfügbare medizinische Daten. Die medizinischen Daten können sowohl medizinische Bilddaten als auch Nicht-Bilddaten umfassen. Medizinische Bilddaten sind dabei Bilddaten, die mit einer bildgebenden Modalität aufgenommen wurden und insbesondere ein Körperteil des Patienten darstellen können. Bildgebende Modalitäten können dabei beispielsweise Computertomografie-Geräte, Magnetresonanzgeräte, Röntgengeräte, Ultraschallgeräte und dergleichen umfassen. Ferner können medizinische Bilddaten digitalisierte Histopathologiebilder umfassen, die einen entsprechend präparierten Gewebeschnitt des Patienten darstellen. Die Bilddaten können ferner longitudinale Daten umfassen, etwa in Form von Zeitserien oder zeitlich beabstandeten Folgeaufnahmen. Nicht-Bilddaten können, insbesondere longitudinale, Daten umfassen, die eine oder mehrere medizinische Werte des Patienten und/oder Elemente aus der Krankheitshistorie des Patienten beinhalten. Dabei kann es sich um Labordaten, Vitalwerte und/oder sonstige auf den Patienten bezogene Messwerte bzw. Voruntersuchungen handeln. Ferner können die Nicht-Bilddaten auf den Patienten bezogene demografische Angaben etwa bezogen das Alter, das Geschlecht, die Lebensgewohnheiten, Risikofaktoren usw. umfassen. Zudem können Nicht-Bilddaten ein oder mehrere Vorbefunde und oder andere Einschätzungen (etwa von anderen, ggf. überweisenden Ärzten) aufweisen. Diese können beispielsweise in Form ein oder mehrerer strukturierter oder unstrukturierter medizinischer Befunde in dem medizinischen Datensatz inkludiert sein.

Der medizinische Datensatz kann nach einigen Ausführungsformen/Aspekten der Erfindung sehr umfassende Informationen über den Gesundheitszustand des Patienten aufweisen (nach anderen Ausführungsformen/Aspekten aber auch nur auf eine Datenkategorie begrenzt sein - etwa Bilddaten und dabei insbesondere Radiologiedaten). Es kann eine Aufgabe des Nutzers sein, basierend auf dem medizinischen Datensatz einen medizinischen Befund bzw. eine medizinische Diagnose oder Schlussfolgerung zu erstellen.

Um den Nutzer dabei zu unterstützen, ist zunächst vorgesehen, anhand des medizinischen Datensatzes durch eine automatische Verarbeitung nach ähnlichen Fällen zu suchen, für die die bereits getroffene Befunde/Diagnosen/Schlussfolgerungen (vor-)bekannt sind. Dem liegt der Gedanke zugrunde, dass Erkenntnisse aus ähnlich gelagerten Fällen für den vorliegenden Fall potentiell relevant sein können. Dazu ist vorgesehen, in einer Menge Vergleichsdatensätze Referenzdatensätze zu identifizieren, welche Referenzdatensätze eine gewisse Ähnlichkeit mit dem medizinischen Datensatz aufweisen. Die Vergleichsdatensätze können eine ähnliche Struktur wie der medizinische Datensatz aufweisen, also insbesondere Bilddaten und Nicht-Bilddaten umfassen. Für die Vergleichsdatensätze ist zudem jeweils wenigstens ein medizinischer Befund vorbekannt. Die vorbekannten medizinischen Befunde können beispielsweise von einem oder mehreren Ärzten verifiziert bzw. annotiert worden sein. Insbesondere können sie von dem Nutzer selbst in der Vergangenheit verifiziert worden sein. Dabei können die Vergleichsdatensätze mit beliebigen medizinischen Befunden assoziiert sein. Alternativ können die medizinischen Befunde aus einer vordefinierten Menge an medizinischen Befunden ausgewählt sein. Die Vergleichsdatensätze können in einer oder mehreren Datenbanken gespeichert sein, die gleichsam Teil medizinischer Informationssysteme sein können. Insbesondere können die Vergleichsdatensätze realen Patientendaten entsprechen, die vorzugweise dahingehend anonymisiert wurden, dass der jeweils zugrundeliegende Patient nicht mehr zuordenbar ist. Alternativ oder zusätzlich können die Vergleichsdatensätze synthetische Datensätze sein, die nicht auf real existierenden Patienten beruhen. Ferner können die Vergleichsdatensätzen aus medizinischen Richtlinien, Kompendien oder Lehrbüchern abgeleitet sein, in denen medizinische Befunde und ihre klinischen Anzeichen prototypisch angegeben sind.

Um die Referenzdatensätze zu bestimmen, können alle zur Verfügung stehenden Vergleichsdatensätze auf ihre Ähnlichkeit zum medizinischen Datensatz untersucht werden. Alternativ kann basierend auf dem medizinischen Datensatz auch eine Vorauswahl aus den Vergleichsdatensätzen getroffen werden, wobei bei der Identifizierung der Referenzdatensätze nur die vorausgewählten Vergleichsdatensätze berücksichtigt werden. Wenn der medizinische Datensatz beispielsweise Histopathologiebilddaten enthält, können dadurch solche Vergleichsdatensätze vorausgewählt werden, die ebenfalls Histopathologiebilddaten enthalten, was die Verarbeitung effizienter gestalten und die Rückmeldungszeit verkürzen kann. Für die berücksichtigten Vergleichsdatensätze kann jeweils ein Ähnlichkeitsmaß bestimmt werden, das auf einer Ähnlichkeit zwischen dem medizinischen Datensatz und dem jeweiligen Vergleichsdatensatz basiert und insbesondere eine Ähnlichkeit angibt bzw. quantifiziert. Ein Ähnlichkeitsmaß kann beispielsweise ein Zahlenwert oder "Score" sein. Die Ähnlichkeitsmaße können beispielsweise basierend auf der Anwendung einer Ähnlichkeitsmetrik bestimmt werden, die basierend auf den Eingangsgrößen, also dem medizinischen Datensatz und einem Vergleichsdatensatz, ein Ähnlichkeitsmaß ausgibt. Die Ähnlichkeitsmetrik kann dabei insbesondere in einem (ersten) Datenverarbeitungsalgorithmus implementiert sein. Referenzdatensätze sind insbesondere solche Vergleichsdatensätze, die eine gewisse Ähnlichkeit mit dem medizinischen Datensatz aufweisen. Mit anderen Worten können Referenzdatensätze insbesondere solche Vergleichsdatensätze sein, deren Ähnlichkeitsmaß über einer vorbestimmten bzw. vorgegeben oder vorgebbaren Schwelle liegt.

Da jeder Vergleichsdatensatz mit wenigstens einem medizinischen Befund assoziiert ist, liefert die automatische Suche nach ähnlichen Datensätzen eine Auswahl medizinscher Befunde, die möglicherweise für den zu befundenden Patienten relevant sind. Darüber hinaus kann aus den Ähnlichkeitsmaßen abgeschätzt werden, welche medizinische Befunde aus dieser Auswahl möglicherweise relevanter sind als andere. Dem liegt der Gedanke zugrunde, dass hohe Ähnlichkeitsmaße eine hohe Relevanz des jeweiligen Vergleichsdatensatz für den vorliegenden Fall indizieren. Optional kann basierend auf den Ähnlichkeitsmaßen deshalb für einen oder mehrere der mit den Referenzdatensätzen assoziierten vorbekannten medizinischen Befunde ein Relevanzwert berechnet werden, der insbesondere auf einer relativen Relevanz des jeweiligen medizinischen Befunds gegenüber anderen medizinischen Befunden basiert, bzw. eine solche angibt und/oder quantifiziert.

Zur weiteren Eingrenzung der möglichen medizinischen Befunde ist vorgesehen, den Nutzer in einer fortgesetzten und geführten Mensch-Maschine-Interaktion einzubinden. Dazu ist vorgesehen, für mögliche medizinische Befunde (also die mit den Referenzdatensätzen assoziierten medizinische Befunde) jeweils ein oder mehrere medizinische Attribute zu identifizieren, die sich durch den Nutzer einfach auf ein Zutreffen auf den vorliegenden Fall überprüfen lassen. Insbesondere können für jeden medizinischen Befund mehrere verschiedene medizinischer Attribute identifiziert werden. Die medizinischen Attribute können dabei übergeordnete Eigenschaften des medinischen Befundes bezeichnen. Mit anderen Worten können die medizinischen Befunde als, insbesondere klinische, Merkmale des jeweiligen medizinischen Befunds aufgefasst werden. Dabei können einem medizinischen Befund mehrere verschiedene medizinische Attribute zugeordnet werden. In Summe können die medizinischen Attribute den ihnen zugeordneten medizinischen Befund charakterisieren und insbesondere von anderen medizinischen Befunden / Differentialdiagnosen abgrenzen. Die medizinischen Attribute können insbesondere so gewählt sein, dass der Nutzer einfach entscheiden kann, ob das medizinische Attribut für den vorliegenden Fall einschlägig ist. Die medizinischen Attribute können in ein oder mehrere Kategorien eingeteilt sein. Insbesondere können die medizinischen Attribute so bestimmt und zugeordnet sein, dass die einem medizinischen Befund zugeordnete Kombination an medizinischen Attributen diesen eindeutig identifizierbar macht. Die identifizierten medizinischen Attribute können dabei beliebig sein und insbesondere dynamisch in Abhängigkeit der mit den Referenzdatensätzen assoziierten medizinischen Befunde festgelegt werden. Alternativ können die medizinischen Attribute im Schritt des Identifizierens aus einer vorbestimmten Menge an medizinischen Attributen ausgewählt werden. Die medizinischen Attribute können auf Grundlage einer vordefinierten und deterministischen Vorschrift identifiziert werden. Die medizinischen Attribute können durch einen Datenverarbeitungsalgorithmus identifiziert werden, der den mit den Referenzdatensätzen assoziierten medizinischen Befunden jeweils ein oder mehrere medizinische Attribute zuordnet. Der Datenverarbeitungsalgorithmus kann beispielsweise als elektronischer Klassifikator ausgebildet sein.

Um dem Nutzer die Möglichkeit einer Überprüfung zu gewähren, ob bzw. welche der identifizierten medizinischen Attribute für den vorliegenden Fall zutreffend sind bzw. in welchem Maß sie zutreffend sind, werden die identifizierten medizinischen Attribute dem Nutzer über eine Nutzerschnittstelle angezeigt. Die Nutzerschnittstelle kann entsprechend ausgebildet sein, beispielsweise indem sie eine grafische Benutzerschnittstelle mit entsprechenden Anzeigefunktionen ausführt. Über die Nutzerschnittstelle kann der Nutzer ferner eine Nutzereingabe betreffend die angezeigten medizinischen Attribute eingeben. Auch dazu kann die Nutzerschnittstelle entsprechend ausgebildet sein, beispielsweise indem sie eine grafische Benutzerschnittstelle mit entsprechenden Eingabefunktionen ausführt.

Da die Nutzereingabe auf die medizinischen Attribute bezogen ist, welche wiederrum Kennzeichen der mit den Referenzdatensätzen assoziierten medizinischen Befunde sind, hat die Nutzereingabe eine mögliche Auswirkung auf die Relevanz der mit den Referenzdatensätzen assoziierten medizinischen Befunde für den zu befundenden Patienten. Dies wird ausgenutzt, um basierend auf dem medizinischen Befunden eine medizinische Information zu erzeugen. Die medizinische Information kann insbesondere eine die Befundung des Patienten betreffende Information sein und beispielsweise angeben, ob ein oder mehrere der medizinischen Befunde für den zu befundenden Patienten relevant sind oder welche medizinischen Befunde ausgeschlossen werden können. Die medizinische Information kann insbesondere angeben, welche der medizinischen Befunde die Nutzereingabe indiziert. Ferner kann die medizinische Information Zusatzinformationen enthalten, die aus dem medizinischen Datensatz und/oder den Referenzdatensätzen entnommen wurden. Diese Zusatzinformationen können beispielsweise auf jenen Datenobjekte in dem medizinischen Datensatz und den Referenzdatensätzen basieren, die als ähnlich identifiziert werden.

Durch den Schritt des Bereitstellens wird die medizinische Information für die weitere Verwendung zur Verfügung gestellt. Beispielsweise kann die medizinische Information zur Darstellung der medizinischen Information an den Nutzer über die Nutzerschnittstelle bereitgestellt werden. Alternativ oder zusätzlich kann die medizinische Information zur Speicherung in einer Speichereinrichtung oder zur weiteren Datenverarbeitung bereitgestellt werden. Insbesondere kann die medizinische Information zur Verbesserung eines Datenverarbeitungsalgorithmus zur Auswahl der Referenzdatensätze oder eines Datenverarbeitungsalgorithmus zur Identifizierung der medizinischen Attribute bereitgestellt werden. Ferner können basierend auf der medizinischen Informationen durch den Nutzer weitere auf die medizinischen Attribute gerichtete Nutzereingaben vorgenommen werden, woraufhin eine neue medizinischen Information ermittelt werden oder eine bestehende medizinische Information angepasst werden kann.

Die Merkmale wirken synergetisch dahingehend zusammen, den Nutzer bei der Stellung einer medizinischen Diagnose zu unterstützen. Mit anderen Worten wird ein Verfahren zur Erstellung einer medizinischen Diagnose durch ein automatisiertes System bereitgestellt, das physiologische Messungen (in Form des medizinischen Datensatzes und der Vergleichsdatensätze) verarbeitet. Dabei wird ein zweistufiger Ansatz verfolgt. Zunächst werden mögliche medizinische Befunde durch eine Ähnlichkeitsanalyse der verfügbaren Daten eingegrenzt. Dies ist die Voraussetzung, den Nutzer bei dem anschließenden Entscheidungsprozess, inwieweit die möglichen medizinischen Befunde für den zu befundenden Patienten relevant sind, durch eine, insbesondere iterative, Mensch-Maschine-Interaktion zu unterstützen. Dabei wird die Entscheidungsfindung durch die automatische Identifizierung von medizinischen Attributen systematisch in für den Nutzer einfacher zu überblickende Teilprobleme zerlegt, da er bei den übergeordneten medizinischen Attributen typischerweise leichter entscheiden kann, ob diese für den vorliegenden Fall zutreffen als das bei einem vollständige Befund der Fall ist. Die medizinischen Attribute sind dabei objektive Merkmale des jeweiligen medizinischen Befundes und mithin von subjektiven Nutzerwahrnehmungen unabhängig. Trotzdem ist das Ergebnis nicht deterministisch oder vorbestimmt, sondern hängt von den spezifischen Nutzereingaben ab. Auf diese Art kann eine verbesserte Aussage darüber herausgearbeitet werden, welche medizinischen Befunde für den zu befundenden Patienten relevant sind, wodurch sowohl die Konfidenz als auch die Genauigkeit der bereitgestellten medizinischen Information aufgabengemäß verbessert werden kann.

Mit anderen Worten werden also Verfahren und entsprechende Vorrichtung zur Bereitstellung einer medizinischen Information offenbart. Dabei werden basierend auf den zur Verfügung stehenden Daten zu einem zu befundenden Patienten zunächst mögliche medizinische Befunde ermittelt. Zu den möglichen medizinischen Befunden werden dann medizinische Attribute identifiziert, welche die möglichen medizinischen Befunde im Kontext des zu befundenen Patienten kennzeichnen. Die medizinischen Attribute werden dann einem Nutzer zur Bewertung/Verifikation angezeigt. Eine entsprechende Nutzereingabe wird dazu verwendet die Relevanz der möglichen medizinischen Befunde zu bewerten.

Gemäß einem weiteren Aspekt wird ein Computer-implementiertes Verfahren zur Bereitstellung einer medizinischen Information bereitgestellt, das mehrere Schritte wie folgt aufweist:
Empfangen eines medizinischen Datensatzes eines Patienten;
Zugreifen auf mehrere Vergleichsdatensätze, welche Vergleichsdatensätze jeweils mit einem vorbekannten medizinischen Befund assoziiert sind;
Berechnen eines Ähnlichkeitsmaßes für jeden Vergleichsdatensatz, wobei jedes Ähnlichkeitsmaß eine Ähnlichkeit zwischen dem jeweiligen Vergleichsdatensatz und dem medizinischen Datensatz angibt;
Identifizieren eines oder mehrerer medizinscher Attribute basierend auf den Ähnlichkeitsmaßen, welche medizinischen Attribute jeweils ein Kennzeichen eines oder mehrerer der vorbekannten medizinischen Befunde umfassen;
Anzeigen der identifizierten medizinischen Attribute über eine Nutzerschnittstelle;
Empfangen einer Nutzereingabe des Nutzers betreffend die angezeigten medizinischen Attribute über die Nutzerschnittstelle;
Erzeugen einer medizinischen Information für den Patienten basierend auf den vorbekannten medizinischen Befunden sowie der Nutzereingabe und/oder den Ähnlichkeitsmaßen; und
Bereitstellen der medizinischen Information.

Das Erzeugen der medizinischen Information umfasst ein Bestimmen einer Rangfolge wenigstens eines Teils der mit den Referenzdatensätzen assoziierten vorbekannten medizinischen Befunde basierend auf den für die jeweiligen Referenzdatensätze bestimmten Ähnlichkeiten und/oder basierend auf der Nutzereingabe.

Mit der Rangfolge wird eine Aussage über die relative Relevanz der medizinischen Befunde für den zu befundenden Patienten bereitgestellt. Dies erlaubt eine differenzierte Beurteilung der Ergebnisse der Verarbeitung (sowohl durch den Nutzer als auch durch nachfolgende Verarbeitungsschritte), wodurch sowohl die Konfidenz als auch die Genauigkeit der bereitgestellten medizinischen Information verbessert werden kann. Die Rangfolge kann dabei ein Berechnen eines Relevanz- oder Konfidenzwertes basierend auf den Ähnlichkeitsmaßen für jeden medizinischen Befunds und/oder den Nutzereingaben umfassen. Dabei können entweder nur die Nutzereingabe oder nur die Ähnlichkeitsmaße berücksichtigt werden. Ferner kann eine Synthese dieser beiden Informationen erfolgend, etwa dadurch, dass auf den Ähnlichkeitsmaßen basierende Relevanz- oder Konfidenzwerte mittels den Nutzereigaben angepasst werden. Nach jeder Iteration der Verfahren, also insbesondere nach jeder auf die identifizierten Attribute gerichteten Nutzereingabe, kann die Rangfolge neu bestimmt werden.

Gemäß einem Aspekt umfasst das Bereitstellen der medizinischen Information ein Anzeigen der medizinischen Information über die Nutzerschnittstelle, wobei das Anzeigen der medizinischen Information basierend auf der Rangfolge und insbesondere in einer Darstellungsform erfolgt, die derart ausgebildet ist, dass der Nutzer die Rangfolge der medizinischen Befunde wenigsten teilweise wahrnehmen kann.

Dies hat den technischen Effekt, dass die relative Relevanz des jeweiligen medizinischen Befundes von dem Nutzer während der fortgesetzten und geführte Mensch-Maschine-Interaktion an den Nutzer kommuniziert und so bei der weiteren Analyse berücksichtigt werden können. Dabei wird der Nutzer in der technischen Aufgabe unterstützt, basierend auf der Analyse von medizinischen Daten eine medizinische Diagnose zu erstellen. Die durch das Merkmal bereitgestellte Unterstützung ist ursächlich mit dem durch die Verarbeitung abgeleiteten objektiven Ergebnis, der medizinischen Information, verknüpft. Die Anzeige dieser Information als solche hängt dabei nicht von den subjektiven Präferenzen ab. Es gibt verschiedene Möglichkeiten, wie die Anzeige basierend auf der Rangfolge und insbesondere die Darstellungsform ausgestaltet sein können. Beispielsweise können die medizinischen Befunde in Form einer Liste mit ab- oder aufsteigender relativer Relevanz angezeigt werden. Daneben ist ein Durchnummerieren der medizinischen Befunde entsprechend ihres Platzes in der Rangfolge und ein Anzeigen der Rangnummer möglich. Ferner kann das Anzeigen ein Anzeigen des vorgenannten Konfidenz- oder Relevanzwertes umfassen. Dies hat den zusätzlichen technischen Effekt, dass der Nutzer Informationen an die Hand bekommt, wie stark sich die relative Relevanz der medizinischen Befunde untereinander unterscheidet. Grundsätzlich können im Schritt des Anzeigens alle oder nur ein Teil (vorzugsweise die relevantesten) medizinischen Befunde angezeigt werden.

Gemäß einem Aspekt umfassen die Verfahren ferner einen Schritt des Bereitstellens einer Zusatzinformation zu ein oder mehreren der medizinischen Befunde und das Anzeigen der medizinischen Information beinhaltet ein Anzeigen der Zusatzinformation.

Die Zusatzinformation kann dabei übergeordnete Information oder Metainformation zu dem jeweiligen medizinischen Befund umfassen, welche beispielsweise aus elektronischen Textbüchern, Richtlinien oder Kompendien bereitgestellt werden kann. Die Zusatzinformation kann beispielsweise Informationen zu Verteilungen der medizinischen Befunde in verschiedenen Kohorten und/oder Informationen zum weiteren Vorgehen umfassen, wie zum Beispiel Behandlungsmöglichkeiten oder weitere Untersuchungsschritte. Damit bekommt der Nutzer automatisch weitere Angaben geliefert, die einerseits das Ergebnis der Analyse transparenter machen können und anderseits weitere Schritte vorschlagen. Damit wird der Nutzer bei der Stellung einer medizinischen Diagnose zielgerichtet unterstützt. Da der Nutzer diese Informationen insbesondere nicht selbst beschaffen muss, kann der Ablauf effizienter und standardisierter gestaltet werden.

Gemäß einem Aspekt kann die Anzeige der Zusatzinformation derart erfolgen, dass die jeweilige Zusatzinformation durch den Nutzer dem jeweiligen medizinischen Befund zuordenbar ist.

Gemäß einem Aspekt umfassen die Verfahren ferner einen Schritt des Extrahierens eines oder mehrerer Datenobjekte aus einem oder mehreren Referenzdatensätzen und/oder dem medizinischen Datensatz und das Anzeigen der medizinischen Information beinhaltet ein Anzeigen der Datenobjekte. Dabei ist bevorzugt, dass die insbesondere Datenobjekte sind, auf deren Grundlage die Ähnlichkeitsmaße bestimmt wurden. Mit anderen Worten können sich die Datenobjekte also auf die Ähnlichkeiten der Referenzdatensätze mit dem medizinischen Datensatz beziehen. Beispielsweise können die Datenobjekte Bilddaten umfassen, die ähnliche krankhafte Veränderung zweigen (insbesondere ähnliche krankhafte Veränderungen wie in den Bilddaten des medizinischen Datensatz). Die Bilddaten können im Schritt des Anzeigens z.B. als (Pixel-)Bilder angezeigt werden.

Durch das Extrahieren und Anzeigen der Datenobjekte kann der Nutzer die Güte der vorgenommenen automatischen Ähnlichkeitsanalyse besser abschätzen und insbesondere die Nutzereingabe auf eine breite Informationsgrundlage aufbauen. Dadurch wird die Mensch-Maschine-Interaktion verbessert.

Gemäß einem Aspekt kann die Anzeige der Datenobjekte derart erfolgen, dass ein jeweiliges Datenobjekt durch den Nutzer dem jeweiligen medizinischen Befund zuordenbar ist. Insbesondere können die Datenobjekte nach den medizinischen Befunden gruppiert sein.

Gemäß einem Aspekt umfassen die medizinischen Attribute eine oder mehrere der folgenden Angaben:
eine, insbesondere demografische, Angabe bezüglich einer mit dem klinischen Befund assoziierten Patientengruppe;
eine Angabe bezüglich eines oder mehrerer mit dem jeweiligen medizinischen Befund assoziierter Symptome;
eine Angabe bezüglich eines oder mehrerer mit dem jeweiligen medizinischen Befund assoziierter Differentialdiagnosen;
eine Angabe bezüglich eines oder mehrerer mit dem jeweiligen medizinischen Befund assoziierter klinischer Anzeichen; und/oder
eine Angabe bezüglich eines oder mehrerer mit dem jeweiligen medizinischen Befund assoziierter klinischer Gegenanzeichen.

Die vorgenannten Angaben können auch als Kategorien der medizinischen Attribute bezeichnet werden. Eine demografische Angabe kann dabei beispielsweise eine Altersgruppe sein, in der ein medizinscher Befund typischerweise auftritt. Ferner kann der eine demografische Angabe ein Geschlecht oder typische Lebensgewohnheiten und/oder Risikofaktoren der mit dem medizinischen Befund typischerweise assoziierten Patientengruppe umfassen und beispielsweise angeben, ob die Patienten Raucher sind. Die klinischen Anzeichen oder Gegenanzeichen können sich dabei unter Anderem auf einen Krankheitsverlauf beziehen und beispielsweise angeben, ob ein medizinischer Befund mit einer akuten, subakuten oder chronischen Erkrankung einhergeht. Ferner können sich klinischen Anzeichen oder Gegenanzeichen auf die Verortung einer krankhaften Veränderung beziehen und beispielsweise angeben, ob Läsionen in der Lunge einseitig, beidseitig oder fokal vorkommen. Ferner können sich klinischen Anzeichen oder Gegenanzeichen auf eine Auftrittshäufigkeit einer medizinischen Diagnose beziehen. Durch die solche medizinischen Attribute können medizinische Befunde multilateral charakterisiert werden und die auf die medizinischen Befunde gerichtete Nutzereingabe erlaubt eine differenzierte Bewertung, wodurch sowohl die Konfidenz als auch die Genauigkeit der bereitgestellten medizinischen Information verbessert werden kann.

Gemäß einem Aspekt umfassen die Verfahren im Schritt des Identifizierens der ein oder mehreren medizinischen Attribute ein Bereitstellen einer Zuordnung, die wenigstens einem vorbekannten medizinischen Befund ein oder mehrere medizinische Attribute zuordnet sowie ein Zuordnen ein oder mehrerer medizinischen Attribute zu wenigstem einem der mit den Referenzdatensätzen assoziierten medizinischen Befunde unter Verwendung der Zuordnung. Insbesondere können im Schritt des Zuordnens jedem mit den Referenzdatensätzen assoziierten medizinischen Befund mehrere verschiedene medizinische Attribute zugeordnet werden.

Die Zuordnung kann insbesondere vordefiniert sein. Beispielsweise kann die Zuordnung in Form einer Liste vorliegen, die jedem medizinischen Befund ein oder mehrere, und insbesondere mehrere verschiedene, medizinische Attribute zuordnet. Die Liste kann beispielsweise in einer Speichereinheit gespeichert sein. Ferner kann die Zuordnung derart ausgestaltet sein, dass die medizinischen Attribute in dem jeweiligen Vergleichsdatensatz entsprechend den jeweils assoziierten medizinischen Befunden gespeichert sind. Durch die Bereitstellung der Zuordnung kann eine rasche und objektive Identifizierung der medizinischen Attribute erfolgen, wodurch die Konfidenz und die Genauigkeit der bereitgestellten medizinischen Information verbessert werden kann.

Der Schritt des Identifizierens der ein oder mehreren medizinischen Attribute umfasst ein Bestimmen einer Attribut-Rangfolge basierend auf den für die jeweiligen Referenzdatensätze bestimmten Ähnlichkeitsmaßen und/oder basierend auf einer diskriminierenden Wirkung der medizinischen Attribute hinsichtlich der Relevanz der medizinischen Befunde für den zu befundenden Patienten und/oder basierend auf einer oder mehrerer verschiedener Attribut-Kategorien, wobei das Anzeigen der identifizierten medizinischen Attribute in einer Darstellungsform erfolgt, die derart ausgestaltet ist, dass der Nutzer die Attribut-Rangfolge wahrnehmen kann.

Dadurch werden dem Nutzer zusätzliche Informationen darüber vermittelt, wie entscheidend ein medizinisches Attribut für die medizinische Information ist. Dadurch wird einerseits das Ergebnis der Analyse für den Nutzer besser nachvollziehbar. Andererseits kann sich der Nutzer auf medizinische Attribute konzentrieren und ggf. seine Nutzereingaben priorisieren, was die Mensch-Maschine-Interaktion effizienter gestalten kann. Die diskriminierende Wirkung kann beispielsweise durch eine Analyse möglicher Auswirkungen auf die Rangfolge oder einer Simulation möglicher Entscheidungsbäume, die zur Bestätigung eines der medizinischen Befunde führen.

Gemäß einem Aspekt kann das Anzeigen der identifizierten medizinischen Attribute in einer derart erfolgen, dass die medizinischen Attribute nach Kategorien gruppiert sind, was dem Nutzer einen besseren Überblick über die medizinischen Attribute verschafft.

Grundsätzlich können alle identifizierten medizinischen Attribute oder nur ein Teil der identifizierten medizinischen Attribute umfassen, wobei bereits der Schritt des Identifizierens ein Auswählen aus den den medizinischen Befunden zugeordneten medizinischen Attributen umfassen kann.

Gemäß einem Aspekt weisen die Verfahren ferner einen Schritt des Erzeugens einer inzidenten medizinischen Information basierend auf den mit den Referenzdatensätzen assoziierten medizinischen Befunden, insbesondere basierend auf den für die jeweiligen Referenzdatensätze bestimmten Ähnlichkeiten, wobei der Schritt des Erzeugens der medizinischen Information ein Anpassen der inzidenten medizinischen Information basierend auf der Nutzereingabe umfasst.

Die inzidente medizinische Information kann im Wesentlich wie oben in Zusammenhang mit der medizinischen Information beschrieben erzeugt werden und insbesondere die gleichen oder ähnliche Bestandteile aufweisen. Die inzidente medizinische Information unterscheidet sich von der medizinischen Information dadurch, dass sie die Nutzereingabe nicht berücksichtigt. Sie basiert mit anderen Worten nur auf der Ähnlichkeitsanalyse. Durch ihr Anpassen basierend auf der Nutzereingabe kann dann die medizinische Information erstellt werden, wodurch die Konfidenz und die Genauigkeit der bereitgestellten medizinischen Information in einem mehrstufigen Prozess verbessert werden kann.

Gemäß einem Aspekt umfassen die Verfahren ferner einen Schritt des Anzeigens der inzidenten medizinischen Information, insbesondere zusammen mit den identifizierten medizinischen Attributen.

Der Nutzer bekommt dadurch eine Aussage über das Ergebnis der Ähnlichkeitsanalyse bereitgestellt, und kann besser abschätzen, auf welcher Grundlage die medizinischen Attribute ausgewählt wurden. Er kann damit seine Nutzereingabe auf eine breitere Informationsgrundlage stellen. Dadurch können die Verfahren anwenderfreundlicher und effizienter werden. Außerdem kann dies das Vertrauen des Nutzers in die Auswertung erhöhen.

Gemäß einem Aspekt weist die Nutzereingabe eine Verifikation wenigstens eines Teils der identifizierten medizinischen Attribute und insbesondere Bewerten eines oder mehrerer der identifizierten Attribute auf. Gemäß weiteren Aspekten weist die Nutzereingabe weiterhin eine Gewichtung eines oder mehrerer der identifizierten medizinischen Attribute auf.

Durch die Aus- oder Abwahl einzelner medizinischen Attribute kann der Nutzer entscheiden, welche der medizinischen Attribute auf den im vorliegenden Fall zutreffen. Durch eine Bewertung ist außerdem eine graduelle Abstufung möglich. Dadurch ergibt sich ein Muster an medizinischen Attributen für den vorliegenden Fall, das mit den Attribut-Muster, der medizinischen Befunde verglichen werden kann, um so relevante medizinische Befunde zu identifizieren und weniger relevante auszuschließen. Mit einer Gewichtung kann der Nutzer beispielsweise zusätzlich angeben, wie entscheidend er das jeweilige medizinische Attribut für die Entscheidungsfindung hält. Für den Nutzer wichtige medizinische Attribute können so beispielsweise gegenüber den anderen medizinischen Attributen bei der Erzeugung bzw. Anpassung der medizinischen Information stärker gewichtet werden als andere (z.B. doppelt). Mit anderen Worten basiert dann der Schritt des Erzeugens der medizinischen Information zusätzlich auf der Gewichtung. Eine Nutzereingabe bezüglich eines medizinischen Attributs mit einer hohen Gewichtung kann dabei einen größeren Einfluss auf die medizinische Information entfalten. Dadurch können die Nutzereingaben noch zielgerichteter ausgebwertet werden, wodurch die Konfidenz und die Genauigkeit der bereitgestellten medizinischen Information verbessert werden können.

Gemäß einem Aspekt umfassen der medizinische Datensatz und die Vergleichsdatensätze jeweils medizinische Bilddaten und ein Ähnlichkeitsmaß basiert jeweils auf einer Ähnlichkeit zwischen den medizinischen Bilddaten des medizinischen Datensatzes und den medizinischen Bilddaten eines Vergleichsdatensatzes.

Mit anderen Worten stellt die Ähnlichkeitsanalyse darauf ab, zu Bildinformation in dem vorliegenden medizinischen Datensatz ähnliche Bilddaten zu finden. Es werden also Bildinformationen miteinander verglichen, um ähnliche Patienten ausfindig zu machen. Es zeigt sich, dass dies ein schneller und akkurater Ansatz ist, die Referenzdatensätze ausfindig zu machen und somit auf möglicherweise relevante medizinische Befunde zu schließen und letztlich die Konfidenz und die Genauigkeit der bereitgestellten medizinischen Information zu verbessern.

Gemäß einem Aspekt umfasst das Ermitteln der ein oder mehrerer Referenzdatensätze die Schritte:
Extrahieren eines Datendeskriptors aus dem medizinischen Datensatz;
Empfangen jeweils eines korrespondierenden Datendeskriptors zu jedem der Vergleichsdatensätze;
Bestimmen, für jeden Vergleichsdatensatz, eines Ähnlichkeitsmaßes, wobei ein Ähnlichkeitsmaß jeweils auf einer Ähnlichkeit zwischen dem Datendeskriptor und einem korrespondierenden Datendeskriptor basiert; und
Ermitteln der ein oder mehrerer Referenzdatensätze basierend auf den bestimmten Ähnlichkeitsmaßen.

Der Datendeskriptor kann ein oder mehrere Merkmale aufweisen, die dem medizinischen Datensatz, und insbesondere den Bilddaten des medizinischen Datensatzes, extrahiert bzw. aus diesen berechnet wurden. Daneben kann der Datendeskriptor basierend auf (oder unter zusätzlicher Berücksichtigung von) weiteren Informationen bzw. Nicht-Bilddaten, wie z.B. Metadaten zu den Bilddaten, Patientendaten, weiteren Messwerten, medizinischen Befunden usw. beruhen. Eine andere Bezeichnung für Datendeskriptor kann der Ausdruck "Merkmalssignatur" sein. Der Datendeskriptor kann insbesondere den medizinischen Datensatz charakterisieren. Die Merkmale des Datendeskriptors können zu einem Merkmalsvektor zusammengefasst sein. Insbesondere kann der Datendeskriptor einen solchen Merkmalsvektor aufweisen. Aus Bilddaten extrahierte Merkmale können morphologische und/oder strukturelle und/oder eine Textur betreffende und/oder ein Muster betreffende Merkmale sein. Aus Nicht-Bilddaten extrahierte können einen Befund, eine medizinischen Bericht, einen Messwert, eine demografische Information usw. betreffende Merkmale sein. Der (erste) Datenverarbeitungsalgorithmus kann insbesondere dazu ausgebildet sein, Ähnlichkeitsmaße basierend auf dem Datendeskriptor zu ermitteln.

Das Ermitteln der Ähnlichkeitsmaße kann ein Extrahieren oder Empfangen eines korrespondierenden Datendeskriptors jeweils aus den möglichen Vergleichsdatensätzen umfassen. Dabei kann wie bei dem basierend auf dem medizinischen Datensatz extrahierten Datendeskriptor vorgegangen werden. Ferner kann das Ermitteln der Ähnlichkeitsmaße ein Vergleichen der korrespondierenden Datendeskriptoren jeweils mit dem Datendeskriptor umfassen. Der Schritt des Vergleichens kann insbesondere auf der Bestimmung eines Abstands der jeweiligen Datendeskriptoren, der Berechnung einer Kosinus-Ähnlichkeit der Datendeskriptoren und/oder der Berechnung einer gewichteten Summe des Unterschieds bzw. der Ähnlichkeit einzelner Merkmale des Datendeskriptoren basieren. Als Referenzdatensätze können insbesondere diejenigen Vergleichsdatensätze identifiziert werden, deren zugehöriges Ähnlichkeitsmaß größer als eine vorgegebene oder vorgebbare Schwelle ist.

Durch die Verwendung von Datendeskriptoren werden einfach zu implementierende und gut übertragbare Parameter für einen Abgleich verschiedener Datensätze definiert. Zudem können die in den Merkmalssignaturen enthaltenen Merkmale auf übergeordneten, aus den Datensätzen abgeleiteten Observablen beruhen, welche die Eigenschaften der Datensätze oftmals besser charakterisieren als die zugrundeliegenden Daten selbst.

Gemäß einem Aspekt umfasst das Ermitteln der ein oder mehreren Referenzdatensätze ein Anwenden einer trainierten Funktion umfasst, welche trainierte Funktion dazu ausgebildet ist, ein Ähnlichkeitsmaß zwischen medizinischen Datensätzen zu bestimmen.

Eine trainierte Funktion bildet allgemein Eingabedaten auf Ausgabedaten ab. Hierbei können die Ausgabedaten insbesondere von einem oder mehreren Parametern der trainierten Funktion abhängen. Der eine oder die mehreren Parameter der trainierten Funktion können durch ein Training bestimmt und/oder angepasst werden. Das Bestimmen und/oder das Anpassen des einen Parameters oder der mehreren Parameter der trainierten Funktion kann insbesondere auf einem Paar aus Trainingseingabedaten und zugehörigen Trainingsausgabedaten basieren, wobei die trainierte Funktion zur Erzeugung von Trainingsabbildungsdaten auf die Trainingseingabedaten angewendet wird. Insbesondere können das Bestimmen und/oder das Anpassen auf einem Vergleich der Trainingsabbildungsdaten und der Trainingsausgabedaten basieren. Im Allgemeinen wird auch eine trainierbare Funktion, d.h. eine Funktion mit noch nicht angepassten Parametern, als trainierte Funktion bezeichnet. Insbesondere kann die trainierte Funktion in dem (ersten) Datenverarbeitungsalgorithmus enthalten sein.

Andere Begriffe für trainierte Funktion sind trainierte Abbildungsvorschrift, Abbildungsvorschrift mit trainierten Parametern, Funktion mit trainierten Parametern, Algorithmus basierend auf künstlicher Intelligenz, Algorithmus des maschinellen Lernens. Ein Beispiel für eine trainierte Funktion ist ein künstliches neuronales Netzwerk. Anstatt des Begriffs "neuronales Netzwerk" kann auch der Begriff "neuronales Netz" verwendet werden. Ein neuronales Netzwerk ist im Grunde genommen wie ein biologisches neuronales Netz - etwa ein menschliches Gehirn - aufgebaut. Insbesondere umfasst ein künstliches neuronales Netzwerk eine Eingabeschicht und eine Ausgabeschicht. Es kann ferner mehrere Schichten zwischen Eingabe- und Ausgabeschicht umfassen. Jede Schicht umfasst mindestens einen, vorzugsweise mehrere, Knoten. Jeder Knoten kann als biologische Verarbeitungseinheit verstanden werden, z.B. als Neuron. Mit anderen Worten entspricht jedes Neuron einer Operation, die auf Eingabedaten angewendet wird. Knoten einer Schicht können durch Kanten oder Verbindungen mit Knoten anderer Schichten verbunden sein, insbesondere durch gerichtete Kanten oder Verbindungen. Diese Kanten oder Verbindungen definieren den Datenfluss zwischen den Knoten des Netzwerks. Die Kanten oder Verbindungen sind mit einem Parameter assoziiert, der häufig als "Gewicht" oder "Kantengewicht" bezeichnet wird. Dieser Parameter kann die Wichtigkeit der Ausgabe eines ersten Knotens für die Eingabe eines zweiten Knotens regulieren, wobei der erste Knoten und der zweite Knoten durch eine Kante verbunden sind. Insbesondere kann eine trainierte Funktion auch ein tiefes künstliches neuronales Netzwerk aufweisen (englischer Fachbegriff sind "deep neural network" oder "deep artificial neural network").

Insbesondere kann ein neuronales Netzwerk trainiert werden. Insbesondere wird das Training eines neuronalen Netzwerks basierend auf den Trainingseingabedaten und den zugehörigen Trainingsausgabedaten gemäß einer "überwachten" Lerntechnik (ein englischer Fachbegriff ist "supervised learning") durchgeführt, wobei die bekannten Trainingseingabedaten in das neuronale Netzwerk eingegeben und die vom Netzwerk generierten Ausgabedaten mit den zugehörigen Trainingsausgabedaten verglichen werden. Das künstliche neuronale Netzwerk lernt und passt die Kantengewichte für die einzelnen Knoten unabhängig an, solange die Ausgabedaten der letzten Netzwerkschicht den Trainingsausgabedaten nicht ausreichend entsprechen.

Gemäß einem Aspekt weist wenigstens eine der trainierten Funktionen ein faltendes neuronales Netzwerk und insbesondere ein bereichsbasiertes faltendes neuronales Netzwerk auf.

Ein englischer Fachbegriff für faltendes neuronales Netzwerk ist convolutional neural network. Insbesondere kann das faltende neuronale Netzwerk als tiefes faltendes neuronales Netzwerk ausgebildet sein (ein englischer Fachbegriff ist "deep convolutional neural network"). Das neuronale Netzwerk weist dabei ein oder mehrere Faltungsschichten (ein englischer Fachbegriff ist "convolutional layer") und ein oder mehrere Entfaltungsschichten (ein englischer Fachbegriff ist "deconvolutional layer") auf. Insbesondere kann das neuronale Netzwerk eine Sammelschicht umfassen (ein englischer Fachbegriff ist "pooling layer"). Durch die Verwendung von Faltungsschichten und/oder Entfaltungsschichten kann ein neuronales Netzwerk besonders effizient zur Bildverarbeitung eingesetzt werden, da trotz vieler Verbindungen zwischen Knotenschichten nur wenige Kantengewichte (nämlich die den Werten des Faltungskerns entsprechenden Kantengewichte) bestimmt werden müssen. Bei einer gleichen Zahl von Trainingsdaten kann damit auch die Genauigkeit des neuronalen Netzwerks verbessert werden.

Ein englischer Fachbegriff für bereichsbasiertes faltendes neuronales Netzwerk ist region-based convolutional neural network. Das bereichsbasierte faltende neuronale Netzwerk kann eine sog. schnelles bereichsbasiertes faltendes neuronales Netzwerk (ein englischer Fachbegriff hierfür ist fast region-based convolutional neural network") oder ein schnelleres bereichsbasiertes faltendes neuronales Netzwerk (ein englischer Fachbegriff hierfür ist fast region-based convolutional neural network") aufweisen. Bereichsbasierte faltende neuronale Netzwerke sind dadurch gekennzeichnet, dass sie integrierte Funktionalitäten zur Definition möglicherweise relevanter Datenbereiche aufweisen, womit sie für eine abschnittsweise Bestimmung von Ähnlichkeiten gemäß Ausführungsformen/Aspekten der Erfindung geeignet sind.

Gemäß einem Aspekt umfasst der Schritt des Bereitstellens ein Bereitstellen der medizinischen Information an die trainierte Funktion und ferner den Schritt des Anpassens der trainierten Funktion basierend auf der medizinischen Information.

In der medizinischen Information umfasst insbesondere eine Nutzereingebe hinsichtlich der medizinischen Attribute. Da ein ähnliches Muster an medizinischen Attributen auf ähnliche medizinische Datensätze hindeutet, kann die medizinische Information dazu verwendet werden, die trainierte Funktion im Einsatz kontinuierlich weiter zu verbessern (ein englischer Ausdruck hierfür ist ,continuous learning').

Gemäß einem Aspekt wird ein System zum Bereitstellen einer medizinischen Information bereitgestellt. Das System weist eine Schnittstelle und eine Steuerung auf. Die Steuerung ist dazu ausgebildet, einen medizinischen Datensatzes eines Patienten über die Schnittstelle zu empfangen. Die Steuerung ist ferner dazu ausgebildet, einen oder mehrere Referenzdatensätze aus mehreren Vergleichsdatensätze basierend auf Ähnlichkeitsmaßen zu ermitteln, wobei ein Ähnlichkeitsmaß auf einer Ähnlichkeit zwischen dem medizinischen Datensatz und einem Vergleichsdatensatz basiert, und wobei jeder Vergleichsdatensatz mit wenigstens einem vorbekannten medizinischen Befund assoziiert ist. Die Steuerung ist ferner dazu ausgebildet, ein oder mehrere medizinische Attribute zu identifizieren, welche medizinischen Attribute jeweils ein Kennzeichen eines oder mehrerer der mit den Referenzdatensätzen assoziierten vorbekannten medizinischen Befunde umfassen. Die Steuerung ist ferner dazu ausgebildet, die identifizierten medizinischen Attribute (über die Schnittstelle anzuzeigen. Die Steuerung ist ferner dazu ausgebildet, eine Nutzereingabe eines Nutzers betreffend die angezeigten medizinischen Attribute über die Schnittstelle zu empfangen. Die Steuerung ist ferner dazu ausgebildet, eine medizinische Information für den Patienten basierend auf den mit den Referenzdatensätzen assoziierten medizinischen Befunden sowie der Nutzereingabe zu erzeugen. Die Steuerung ist ferner dazu ausgebildet, die medizinische Information über die Schnittstelle bereitzustellen.

Die Steuerung kann als zentrale oder dezentrale Recheneinheit ausgebildet sein. Die Recheneinheit kann einen oder mehrere Prozessoren aufweisen. Die Prozessoren können als zentrale Verarbeitungseinheit (ein englischer Fachausdruck hierfür ist "central processing unit", kurz CPU) und/oder als Grafikprozessor (ein englischer Fachausdruck hierfür ist "graphics processing unit", kurz GPU) ausgebildet sein. Alternativ kann die Steuerung als lokaler oder Cloud-basierter Verarbeitungsserver implementiert sein. Ferner kann die Steuerung ein oder mehrere virtuelle Maschinen umfassen.

Die Schnittstelle kann allgemein zum Datenaustausch zwischen der Steuerung und weiteren Komponenten ausgebildet sein. Die Schnittstelle kann in Form von einer oder mehreren einzelnen Datenschnittstellen implementiert sein, welche ein Hardware- und/oder Software-Interface, z.B. einen PCI-Bus, eine USB-Schnittstelle, eine Fire-Wire-Schnittstelle, eine ZigBee- oder eine Bluetooth-Schnittstelle aufweisen können. Die Schnittstelle kann ferner eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle oder eine Wireless LAN-Schnittstelle (WLAN oder Wi-Fi) aufweisen. Die Schnittstelle kann ferner zur Kommunikation mit dem Nutzer über eine Nutzerschnittstelle ausgebildet sein. Entsprechend kann die Steuerung dazu ausgebildet sein, die medizinischen Attribute über die Nutzerschnittstelle anzuzeigen und die Nutzereingabe über die Nutzerschnittstelle zu empfangen.

Die Vorteile der vorgeschlagenen Vorrichtung entsprechen im Wesentlichen den Vorteilen des vorgeschlagenen Verfahrens. Merkmale, Vorteile oder alternative Ausführungsformen/Aspekte können ebenso auf die anderen beanspruchten Gegenstände übertragen werden und umgekehrt.

Gemäß einem Aspekt weist das System ferner eine Datenbank zur Speicherung mehrerer Vergleichsdatensätze und auf. Die Schnittstelle ist in Datenverbindung mit der Datenbank. Die Steuerung ist ferner dazu ausgebildet, zur Bestimmung der Referenzdatensätze auf die Vergleichsdatensätze zuzugreifen.

Die Steuerung ist ferner dazu ausgebildet, Vergleichsdatensätze aus der Datenbank basierend auf dem medizinischen Datensatz auszuwählen.

Die Datenbank kann als zentrale oder dezentrale Speichereinheit ausgebildet sein. Die Datenbank kann insbesondere Teil eines Serversystems sein. Die Datenbank kann insbesondere Teil eines medizinischen Informationssystems wie etwa eines Krankenhausinformationssystem (ein englischer Fachbegriff hierfür ist hospital information system oder kurz HIS) und/der eines PACS-Systems sein (PACS steht dabei für picture archiving and communication system) und/oder eines Labor-Informationssystem (LIS) sein. Die Datenbank kann ferner als sog. Cloud-Speicher ausgebildet sein.

Gemäß einem weiteren Aspekt wird ein Befundungssystem bereitgestellt, welches das System zur Bereitstellung einer medizinischen Information sowie ein medizinischen Informationssystem umfasst, das zur Speicherung und/oder Bereitstellung medizinischer Daten (insbesondere des medizinischen Datensatzes und der Vergleichsdatensätze) ausgebildet ist. Dabei steht das medizinische Information mit dem System zur Bereitstellung einer medizinischen Information über die Schnittstelle in Verbindung. Ferner kann das medizinische Informationssystem ein oder mehrere bildgebende Modalitäten, wie etwa ein Computertomografiesystem, ein Magnetresonanzsystem, ein Angiografiesystem, ein Röntgensystem, ein Positronen-Emissions-Tomografie-System, ein Mammografiesystem, und/oder eine System zur Erzeugung von Histopathologiebilddaten, umfassen.

Die Erfindung betrifft in einem weiteren Aspekt ein Computerprogrammprodukt, das ein Programm umfasst und direkt in einen Speicher einer programmierbaren Steuerung ladbar ist und Programmmittel, z.B. Bibliotheken und Hilfsfunktionen, aufweist, um ein Verfahren zur Bereitstellung einer Ähnlichkeitsinformation insbesondere gemäß den vorgenannten Ausführungsformen/Aspekten auszuführen, wenn das Computerprogrammprodukt ausgeführt wird.

Ferner betrifft die Erfindung in einem weiteren Aspekt ein computerlesbares Speichermedium, auf welchem lesbare und ausführbare Programmabschnitte gespeichert sind, um alle Schritte eines Verfahrens zur Bereitstellung einer Ähnlichkeitsinformation gemäß den vorgenannten Ausführungsformen/Aspekten auszuführen, wenn die Programmabschnitte von dem der Steuerung ausgeführt werden.

Die Computerprogrammprodukte können dabei eine Software mit einem Quellcode, der noch kompiliert und gebunden oder der nur interpretiert werden muss, oder einen ausführbaren Softwarecode umfassen, der zur Ausführung nur noch in die Verarbeitungseinheit zu laden ist. Durch die Computerprogrammprodukte können die Verfahren schnell, identisch wiederholbar und robust ausgeführt werden. Die Computerprogrammprodukte sind so konfiguriert, dass sie mittels der Recheneinheit die erfindungsgemäßen Verfahrensschritte ausführen können. Die Recheneinheit muss dabei jeweils die Voraussetzungen wie beispielsweise einen entsprechenden Arbeitsspeicher, einen entsprechenden Prozessor, eine entsprechende Grafikkarte oder eine entsprechende Logikeinheit aufweisen, sodass die jeweiligen Verfahrensschritte effizient ausgeführt werden können.

Die Computerprogrammprodukte sind beispielsweise auf einem computerlesbaren Speichermedium gespeichert oder auf einem Netzwerk oder Server hinterlegt, von wo sie in den Prozessor der jeweiligen Recheneinheit geladen werden können, der mit der Recheneinheit direkt verbunden oder als Teil der Recheneinheit ausgebildet sein kann. Weiterhin können Steuerinformationen der Computerprogrammprodukte auf einem computerlesbaren Speichermedium gespeichert sein. Die Steuerinformationen des computerlesbaren Speichermediums können derart ausgebildet sein, dass sie bei Verwendung des Datenträgers in einer Recheneinheit ein erfindungsgemäßes Verfahren durchführen. Beispiele für computerlesbaren Speichermedium sind eine DVD, ein Magnetband oder ein USB-Stick, auf welchem elektronisch lesbare Steuerinformationen, insbesondere Software, gespeichert ist. Wenn diese Steuerinformationen von dem Datenträger gelesen und in eine Recheneinheit gespeichert werden, können alle erfindungsgemäßen Ausführungsformen/Aspekte der vorab beschriebenen Verfahren durchgeführt werden. So kann die Erfindung auch von dem besagten computerlesbaren Medium und/oder dem besagten computerlesbaren Speichermedium ausgehen. Die Vorteile der vorgeschlagenen Computerprogrammprodukte bzw. der zugehörigen computerlesbaren Medien entsprechen im Wesentlichen den Vorteilen der vorgeschlagenen Verfahren.

Weitere Besonderheiten und Vorteile der Erfindung werden aus den nachfolgenden Erläuterungen von Ausführungsbeispielen anhand von schematischen Zeichnungen ersichtlich. In diesem Zusammenhang genannte Modifikationen können jeweils miteinander kombiniert werden, um neue Ausführungsformen auszubilden. In unterschiedlichen Figuren werden für gleiche Merkmale die gleichen Bezugszeichen verwendet.

Es zeigen:
Fig. 1 eine schematische Darstellung einer Ausführungsform eines Systems zur Bereitstellung einer medizinischen Information,
Fig. 2 ein Ablaufdiagramm eines Verfahrens zur Bereitstellung einer medizinischen Information gemäß einer Ausführungsform,
Fig. 3 Abhängigkeiten verschiedener Datensätze und Informationen gemäß einer Ausführungsform,
Fig. 4 Abhängigkeiten verschiedener Datensätze und Informationen gemäß einer Ausführungsform,
Fig. 5 ein Ablaufdiagramm eines Verfahrens zur Bereitstellung einer medizinischen Information gemäß einer Ausführungsform,
Fig. 6 ein Ablaufdiagramm eines Verfahrens zur Bereitstellung einer trainierten Funktion gemäß einer Ausführungsform,
Fig. 7 eine grafische Benutzerschnittstelle für ein Verfahren zur Bereitstellung einer medizinischen Information gemäß einer Ausführungsform,
Fig. 8 eine grafische Benutzerschnittstelle für ein Verfahren zur Bereitstellung einer medizinischen Information gemäß einer Ausführungsform, und
Fig. 9 eine grafische Benutzerschnittstelle für ein Verfahren zur Bereitstellung einer medizinischen Information gemäß einer Ausführungsform.

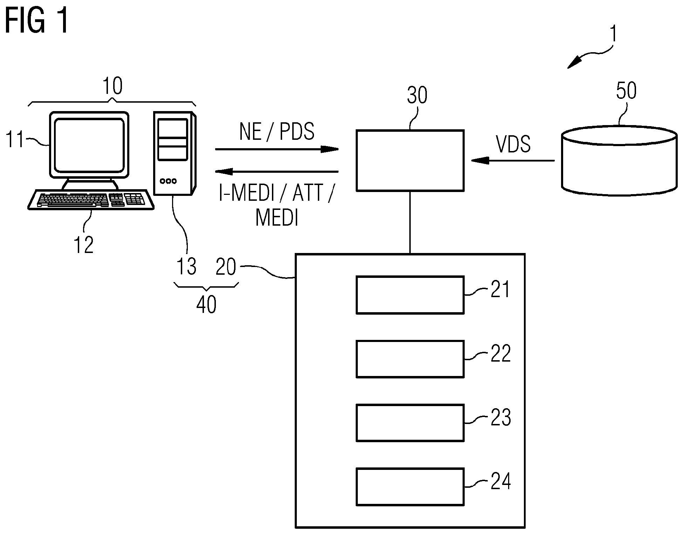

In Figur 1 ist ein System 1 zur Bereitstellung einer medizinischen Information MEDI basierend auf einem medizinischen Datensatz PDS eines Patienten und auf medizinischen Vergleichsdatensätzen VDS gemäß einer Ausführungsform dargestellt. Das System 1 weist eine Nutzerschnittstelle 10, eine Recheneinheit 20, eine Schnittstelle 30 und eine Speichereinheit 50 auf. Die Recheneinheit 20 ist grundsätzlich zur Erzeugung und Bereitstellung der medizinischen Information MEDI basierend auf dem medizinischen Datensatz PDS und den Vergleichsdatensätzen VDS ausgebildet. Die Vergleichsdatensätze VDS können der Recheneinheit 20 über die Schnittstelle 30 von der Speichereinheit 50 bereitgestellt werden. Der medizinische Datensatz PDS kann der Recheneinheit 20 über die Schnittstelle 30 und/oder über die Nutzerschnittstelle 10 bereitgestellt werden (z.B. durch "Hochladen" des medizinischer Datensatz PDS). Alternativ kann die Nutzerschnittstelle 10 der Recheneinheit 20 eine Dateninformation über den medizinischen Datensatz PDS bereitstellen und die Recheneinheit kann den medizinischen Datensatz PDS unter Verwendung der Dateninformation von einer entsprechenden Datenbank (nicht dargestellt) laden. Gemäß einigen Ausführungsformen kann diese Datenbank Teil der Speichereinheit 50 sein.

Die Speichereinheit 50 kann als zentrale oder dezentrale Datenbank ausgebildet sein. Die Speichereinheit 50 kann insbesondere Teil eines Serversystems sein. Die Speichereinheit 50 kann insbesondere Teil eines medizinischen Informationssystems wie etwa eines Krankenhausinformationssystems (ein englischer Fachbegriff hierfür ist hospital information system oder kurz HIS), eines PACS-Systems (PACS steht dabei für picture archiving and communication system) und/oder eines Labor-Informationssystems (LIS) sein. Die Speichereinheit 50 kann ferner als sog. Cloud-Speicher ausgebildet sein. Der Speichereinheit 50 ist dazu ausgelegt, eine Anzahl von Vergleichsdatensätzen VDS zu speichern. Vergleichsdatensätze VDS betreffen Vergleichsfälle mit bekannten oder verifizierten medizinischen Befunden MD1, MD2, MD3. Die Vergleichsdatensätze VDS können das gleiche Format wie der medizinische Datensatz PDS des zu befundenden Patienten aufweisen.

Der medizinische Datensatz PDS stellt die Patientendaten des zu befundenden Patienten dar. Die Vergleichsdatensätze VDS sind Patientendaten von realen und/oder virtuellen/synthetischen Vergleichspatienten (die von dem zu befundenden Patienten verschieden sind). Der medizinische Datensatz PDS sowie die Vergleichsdatensätze VDS können medizinische Bilddaten und/oder andere, keine Bildinformation umfassende, medizinische Daten aufweisen. Bilddaten können sich in diesem Zusammenhang auf medizinische Bilddaten mit zwei oder drei räumlichen Dimensionen beziehen. Ferner können die Bilddaten zusätzlich eine zeitliche Dimension aufweisen. Die Bilddaten können beispielsweise mit einer bildgebenden medizinischen Modalität erzeugt worden sein, wie etwa einem Röntgen-, Computertomografie-, Magnetresonanz-, Positronen-Emissions-Tomografie- oder Angiografie-Gerät oder weiteren Geräten. Solche Bilddaten können auch als Radiologie-Bilddaten bezeichnet werden.

Ferner können der medizinischer Datensatz PDS und/oder die Vergleichsdatensätze VDS auch Histopathologiebilddaten umfassen, die jeweils ein oder mehrere Histopathologiebilder zeigen. Histopathologiebilddaten sind Bilddaten, die auf einer Gewebeprobe eines Patienten beruhen. Aus der Gewebeprobe werden Gewebeschnitte präpariert, die mit einer histologischen Färbung angefärbt wurden. Die so präparierten Gewebeschnitte werden dann digitalisiert, um die Histopathologiebilddaten zu erhalten. Hierfür können spezialisierte Scanner, sog. Slide Scanner, verwendet werden. Das dabei aufgenommene Bild wird auch als "Whole Slide Image" bezeichnet. Die dabei aufgenommen Bilddaten sind typischerweise zweidimensionale Pixeldaten.

Die in dem medizinischer Datensatz PDS oder den Vergleichsdatensätzen VDS enthaltenen Bilddaten können beispielsweise entsprechend dem DICOM-Format formatiert sein. DICOM (=Digital Imaging and Communications in Medicine) ist ein offener Standard für die Kommunikation und Administration von medizinischen Bilddaten und zugehörigen Daten.

Neben Bilddaten können der medizinischer Datensatz PDS und die Vergleichsdatensätze VDS auch Nicht-Bilddaten umfassen. Nicht-Bilddaten können z.B. Untersuchungsergebnisse sein, die nicht auf medizinscher Bildgebung beruhen. Dies kann Labordaten, Vitaldaten, Spirometriedaten oder die Protokolle neurologischer Untersuchungen umfassen. Daneben können Nicht-Bilddaten Textdatensätze, wie etwa strukturierte und unstrukturierte medizinische Befundberichte (ein englischer Begriff hierfür lautet "medical report"), umfassen. Nicht-Bilddaten können ferner auch patientenbezogene Daten sein. Dies kann z.B. demografische Angaben zum Patienten, etwa betreffend sein Alter, das Geschlecht oder das Körpergewicht, umfassen. Die Nicht-Bilddaten können in die Bilddaten z.B. als Metadaten eingebunden sein. Alternativ oder ergänzend können die Nicht-Bilddaten auch in einer elektronischen Krankenakte (ein englischer Ausdruck hierfür ist "Electronic Medical Record" oder kurz EMR) des Patienten, d.h. separat von den Bilddaten, hinterlegt sein. Solche elektronische Krankenakten können beispielsweise in der Speichereinrichtung 50 oder in einer separat hiervon eingerichteten Speichereinrichtung archiviert sein, mit der die Recheneinheit 20 über die Schnittstelle 30 in Verbindung stehen kann.

Die Nutzerschnittstelle 10 kann eine Anzeigeeinheit 11 und eine Eingabeeinheit 12 aufweisen. Die Nutzerschnittstelle 10 kann als tragbares Rechnersystem, wie etwa als Smartphone, Tablet-Computer, oder Laptop ausgebildet sein. Ferner kann die Nutzerschnittstelle 10 als Desktop-PC ausgebildet sein. Die Eingabeeinheit 12 kann in die Anzeigeeinheit 11 integriert sein, beispielsweise in Form eines berührungsempfindlichen Bildschirms. Als Alternative dazu oder zusätzlich kann die Eingabeeinheit 12 eine Tastatur oder eine Computermaus und/oder einen digitalen Stift aufweisen. Die Anzeigeeinheit 11 ist dazu ausgebildet, einzelne oder mehrere Elemente aus dem medizinische Datensatz PDS bzw. den Vergleichsdatensätzen VDS, die ermittelte medizinische Information MEDI sowie für die Bereitstellung der medizinische Information MEDI relevante Schritte insbesondere grafisch darzustellen. Die Nutzerschnittstelle 10 ist ferner dazu ausgebildet, von dem Nutzer eine Eingabe hinsichtlich der medizinische Information MEDI zu erhalten, der für eine Befundung relevant ist. Der Nutzer kann dabei eine Ärztin oder ein Arzt sein.

Die Nutzerschnittstelle 10 weist einen oder mehrere Prozessoren 13 auf, die dazu ausgebildet sind, eine Software zur Ansteuerung der Anzeigeeinheit 11 und der Eingabeeinheit 12 auszuführen, um eine grafische Benutzerschnittstelle GUI bereitzustellen, die es dem Nutzer ermöglicht, einen Patienten bzw. den zugehörigen medizinischen Datensatz PDS für eine Befundung auszuwählen, Nutzereingaben NE zur Bereitstellung der medizinischen Information MEDI in das System 1 einzugeben und die gefundene medizinische Information MEDI zu begutachten. Der Nutzer kann die Software beispielsweise über die Nutzerschnittstelle 10 aktivieren, beispielsweise, indem er sie von einem App-Store herunterlädt und/oder lokal ausführt. Gemäß weiteren Ausführungsformen kann die Software auch ein Client-Server Computerprogramm in Form einer Web-Applikation sein, die in einem Browser läuft.

Die Schnittstelle 30 kann ein oder mehrere einzelne Datenschnittstellen aufweisen, die den Datenaustausch zwischen den Komponenten 10, 20, 50 des Systems 1 gewährleisten. Die ein oder mehreren Datenschnittstellen können Teil der Nutzerschnittstelle 10, der Recheneinheit 20 und/oder der Speichereinheit 50 sein. Die ein oder mehreren Datenschnittstellen können ein Hardware- und/oder Software-Interface, z.B. einen PCI-Bus, eine USB-Schnittstelle, eine Fire-Wire-Schnittstelle, eine ZigBee- oder eine Bluetooth-Schnittstelle aufweisen. Die ein oder mehreren Datenschnittstellen können eine Schnittstelle eines Kommunikationsnetzwerks aufweisen, wobei das Kommunikationsnetzwerk ein Local Area Network (LAN), beispielsweise ein Intranet oder ein Wide Area Network (WAN) aufweisen kann. Entsprechend können die ein oder mehreren Datenschnittstellen eine LAN-Schnittstelle und/oder eine Wireless LAN-Schnittstelle (WLAN oder Wi-Fi) aufweisen.

Die Recheneinheit 20 kann einen Prozessor aufweisen. Der Prozessor kann eine Central Processing Unit (CPU), eine Graphics Processing Unit (GPU), einen digitalen Signalprozessor (DSP), einen Bildverarbeitungsprozessor, einen integrierten (digitalen oder analogen) Schaltkreis oder Kombinationen der vorgenannten Komponenten und weitere Einrichtungen zur Bereitstellung der medizinischen Information MEDI gemäß Ausführungsformen der Erfindung aufweisen. Die Recheneinheit 20 kann als einzelne Komponente implementiert sein oder mehrere Komponenten aufweisen, die parallel oder seriell arbeiten. Alternativ kann die Recheneinheit 20 eine reale oder virtuelle Gruppe von Rechnern aufweisen, wie etwa ein Cluster oder eine Cloud. Ein solches System kann Server-System genannt werden. Je nach Ausführungsform kann die Recheneinheit 20 als lokaler Server oder als Cloudserver ausgebildet sein. Ferner kann die Recheneinheit 20 einen Arbeitsspeicher, wie einen RAM, aufweisen, um beispielsweise den medizinischer Datensatz PDS oder die Vergleichsdatensätze VDS temporär zu speichern. Alternativ kann ein solcher Arbeitsspeicher auch in der Nutzerschnittstelle 10 angeordnet sein. Die Recheneinheit 20 ist z.B. durch computerlesbare Instruktionen, durch Design und/oder Hardware derart ausgebildet, dass sie ein oder mehrere Verfahrensschritte gemäß Ausführungsformen der vorliegenden Erfindung ausführen kann. Insbesondere kann die Recheneinheit 20 dazu ausgebildet sein, ein oder mehrere weiter unten beschriebene trainierte Funktionen auszuführen.

Die Recheneinheit 20 kann Untereinheiten oder Module 21-24 aufweisen, die dazu ausgebildet sind, einem Nutzer im Rahmen einer fortgesetzten Mensch-Maschine Interaktion eine medizinische Information MEDI zur Verfügung zu stellen und ihn so bei der Befundung zu unterstützen.

Modul 21 ist zur Bereitstellung von Referenzdatensätzen Referenzdatensätze RDS-1, RDS-2, ..., RDS-n ausgebildet. Modul 21 kann als Ähnlichkeitsanalyse-Modul bezeichnet werden. Dazu kann Modul 21 dazu ausgebildet sein, auf die Speichereinheit 50 zuzugreifen und aus den Vergleichsdatensäten VDS ein oder mehrere Referenzdatensätze RDS-1, RDS-2, ..., RDS-n auszuwählen. Die Referenzdatensätze RDS-1, RDS-2, ..., RDS-n zeichnen sich dabei dadurch aus, dass sie eine gewisse Ähnlichkeit zu dem medizinischen Datensatz PDS aufweisen. Hierfür kann Modul 21 beispielsweise dazu ausgebildet sein, einen Datendeskriptor aus dem medizinischen Datensatz PDS zu extrahieren und mit entsprechenden Datendeskriptoren aus den Vergleichsdatensätzen VDS zu vergleichen. Ein Datendeskriptor kann dabei als Datenvektor oder Merkmalsvektor aufgefasst werden, in dem relevante Merkmale für Vergleiche verschiedener medizinischer Datensätze aggregiert sind. Solche relevanten Merkmale können dabei sowohl aus den Bilddaten als auch aus den Nicht-Bilddaten des medizinischen Datensatzes PDS extrahiert werden, oder beispielsweise demografische Informationen zum jeweiligen Patienten (wie Alter oder Geschlecht), Laborwerte (wie etwa ein PSA-Wert des Patienten), und/oder Vitaldaten des Patienten umfassen. Aus Bilddaten extrahierte Merkmale können beispielsweise Bildinformationen wie Muster, Farbinformationen, Intensitätswerte usw. umfassen. Der aus dem medizinischen Datensatz PDS erzeugte Datendeskriptor kann von dem Modul 21 mit entsprechenden Datendeskriptoren der Vergleichsdatensätze VDS verglichen werden, um unter den Vergleichsdatensätze VDS Fälle zu ermitteln, die eine gewisse Ähnlichkeit mit dem medizinischen Datensatz PDS des zu befundenden Patienten aufweisen. Dazu kann das Modul 21 für die betrachteten Vergleichsdatensätze VDS jeweils ein Ähnlichkeitsmaß AE-1, AE-2, ..., AE-n ermitteln, das eine Ähnlichkeit des medizinischen Datensatz PDS mit dem jeweiligen Vergleichsdatensätzen VDS angibt (bzw. quantifiziert). Vergleichsdatensätze VDS mit einem gewissen Ähnlichkeitsmaß AE-1, AE-2, ..., AE-n (z.B. einem Ähnlichkeitsmaß AE-1, AE-2, ..., AE-n oberhalb eines gewissen Schwellenwerts) werden als Referenzdatensätze RDS-1, RDS-2, ..., RDS-n identifiziert. Zur Durchführung der Ähnlichkeitsanalyse kann das Modul 21 dazu ausgebildet sein, einen ersten Datenverarbeitungsalgorithmus ALG-1 auszuführen. Der erste Datenverarbeitungsalgorithmus ALG-1 ist dann dazu ausgebildet, basierend auf dem medizinischen Datensatz PDS und den Vergleichsdatensätzen VDS ein oder mehrere Referenzdatensätze RDS-1, RDS-2, ..., RDS-n zu identifizieren und entsprechende Ähnlichkeitsmaße AE-1, AE-2, ..., AE-n bereitzustellen. Der erste Datenverarbeitungsalgorithmus ALG-1 kann ein oder mehrere trainierte Funktionen aufweisen.

Modul 22 ist dazu ausgebildet, basierend auf der Ähnlichkeitsanalyse aus Modul 21 einen Satz medizinischer Attribute medizinischen Attribute ATT-1, ATT-2, ..., ATT-m zu bestimmen. Modul 22 kann als Klassifikator-Modul bezeichnet werden. Dazu können jedem der mit den Referenzdatensätze RDS-1, RDS-2, ..., RDS-n assoziierten vorbekannten medizinischen Befunde MD1, MD2, MD3 jeweils möglichst ein oder mehrere medizinische Attribute ATT-1, ATT-2, ..., ATT-m zugeordnet werden. Hierfür kann das Modul 22 dazu ausgebildet sein, auf eine feste Zuordnung oder Zuordnungsvorschrift zurückzugreifen, die jedem möglichen medizinischen Befund MD1, MD2, MD3 ein oder mehrere verschiedene medizinische Attribute ATT-1, ATT-2, ..., ATT-m zuordnet. Die für die mit den Referenzdatensätzen RDS-1, RDS-2, ..., RDS-n assoziierten medizinischen Befunde MD1, MD2, MD3 ermittelten medizinischen Attribute ATT-1, ATT-2, ..., ATT-m bilden den Satz medizinischer Attribute ATT-1, ATT-2, ..., ATT-m, der nachfolgend dem Nutzer angezeigt wird. Ein medizinisches Attribute ATT-1, ATT-2, ..., ATT-m kann sich dabei jeweils auf ein Kennzeichen, eine Eigenschaft, einen Begleitumstand oder eine (notwendige) Bedingung des jeweiligen Befundes beziehen, zu dem es zugeordnet ist. Ein medizinisches Attribut ATT-1, ATT-2, ..., ATT-m kann auf ein oder mehrere der vorbekannten medizinischen Befunde MD1, MD2, MD3 zutreffen. Ein medizinisches Attribut ATT-1, ATT-2, ..., ATT-m kann somit mit anderen Worten beispielsweise auf eine erste Teilmenge der den Referenzdatensätze RDS-1, RDS-2, ..., RDS-n zugeordneten medizinische Befunde MD1, MD2, MD3 zutreffen, während es auf eine zweite Teilmenge der den Referenzdatensätze RDS-1, RDS-2, ..., RDS-n zugeordneten medizinischen Befunde MD1, MD2, MD3 nicht zutrifft oder diese sogar ausschließt. Kann der Nutzer dieses medizinischen Attribute ATT-1, ATT-2, ..., ATT-m für den zu befundenden Patienten bestätigen, werden die vorbekannten medizinischen Befunde MD1, MD2, MD3 der ersten Teilmenge für den vorliegenden Fall folglich relevanter, während die vorbekannten medizinischen Befunde MD1, MD2, MD3 der zweiten Teilmenge folglich weniger relevant werden oder sogar ausgeschlossen werden können. Umgekehrt können die medizinischen Befunde MD1, MD2, MD3 der ersten Teilmenge ausgeschlossen werden, wenn der Nutzer die medizinischen Attribute ATT-1, ATT-2, ..., ATT-m für den zu befundenden Patienten ausschließen kann. Ein medizinisches Attribut ATT-1, ATT-2, ..., ATT-m kann dabei beispielsweise ein semantisches Kennzeichen, wie beispielsweise ein Symbol, ein Wort oder ein (eine bevorzugt kurze) Wortfolge sein. Ausschließen oder bestätigen kann der Nutzer die Attribute ATT-1, ATT-2, ..., ATT-m durch eine entsprechende Nutzereingabe NE (über Modul 24), wodurch rasch eine weitere Eingrenzung der aufgrund der Ähnlichkeitsanalyse indizierten medizinischen Befunde gelingen kann.

Das Modul 23 ist dazu ausgebildet, basierend auf den zur Verfügung stehenden Eingangsgrößen eine medizinische Information I-MEDI/MEDI zu ermitteln bzw. anzupassen, die den Nutzer bei der Erstellung einer medizinischen Diagnose unterstützt. Modul 23 kann daher als Informations-Erzeugungsmodul bezeichnet werden. Zunächst sind die zur Verfügung stehenden Eingangsgrößen die Ähnlichkeitsmaße AE-1, AE-2, ..., AE-n der Referenzdatensätze RDS-1, RDS-2, ..., RDS-n und die den Referenzdatensätze RDS-1, RDS-2, ..., RDS-n zugeordneten vorbekannten medizinischen Befunde MD1, MD2, MD3. Durch die fortgesetzte Nutzer-System-Interaktionen kommen als weitere Eingangsgrößen die medizinischen Attribute ATT-1, ATT-2, ..., ATT-m und die diesbezüglichen Nutzereingaben NE dazu. Eine zunächst nur auf Grundlage der Ähnlichkeitsanalyse, also den Ähnlichkeitsmaßen AE-1, AE-2, ..., AE-n und den vorbekannten medizinischen Befunde MD1, MD2, MD3, erzeugte medizinische Information wird nachfolgend auch inzidente medizinische Information I-MEDI genannt. Durch die Zuordnung der medizinischen Attribute ATT-1, ATT-2, ..., ATT-m und die Berücksichtigung der entsprechenden Nutzereingaben NE kann diese inzidente medizinische Information I-MEDI angepasst werden und schließlich als medizinische Information MEDI bereitgestellt werden. Das Modul 23 kann in diesem Zusammenhang beispielsweise dazu ausgebildet sein, z.B. basierend auf den Ähnlichkeitsmaßen AE-1, AE-2, ..., AE-n eine Rangfolge der vorbekannten medizinischen Befunde MD1, MD2, MD3 zu erstellen und diese als medizinische Information MEDI auszugeben. Das Modul 23 kann ferner dazu ausgebildet sein, die Rangfolge als Folge der Nutzereingabe NE anzupassen, wenn aufgrund des Zutreffens oder Nicht-Zutreffens einiger medizinischen Attribute ATT-1, ATT-2, ..., ATT-m anzupassen. Das Modul 23 kann dazu ausgebildet sein, eine Anpassung der medizinischen Information MEDI wiederholt durchzuführen, wenn eine neue Nutzereingabe NE vorliegt. Zur Ermittlung der medizinischen Information I-MEDI/MEDI kann das Modul 23 dazu ausgebildet sein, einen zweiten Datenverarbeitungsalgorithmus ALG-2 zu implementieren. Der zweite Datenverarbeitungsalgorithmus ALG-2 ist dazu ausgebildet, basierend auf den medizinischen Befunden MD1, MD2, MD3 sowie den Ähnlichkeitsmaßen AE-1, AE-2, ..., AE-n und/oder der Nutzereingabe NE eine medizinische Information I-MEDI/MEDI zu erzeugen. Insbesondere kann der zweite Datenverarbeitungsalgorithmus ALG-2 als elektronischer Klassifikator implementiert sein. Weiterhin kann der zweite Datenverarbeitungsalgorithmus ein oder mehrere trainierte Funktionen aufweisen. Ferner kann das Modul 23 dazu ausgebildet sein, den medizinischen Datensatz PDS des zu befundenden Patienten automatisch dahingehend zu analysieren, ob eines oder mehrere der medizinischen Attribute ATT-1, ATT-2, ..., ATT-m zutrifft oder nicht zutrifft.

Das Modul 24 kann als Nutzerinteraktions- und/oder Visualisierungsmodul aufgefasst werden. Das Modul 24 ist dazu ausgebildet, dem Nutzer die medizinische Information MEDI und/oder die inzidente medizinische Information I-MEDI sowie die ermittelten medizinischen Attribute ATT-1, ATT-2, ..., ATT-m anzuzeigen. Das Modul 24 ist ferner dazu ausgebildet, eine Nutzereingabe NE bezüglich der medizinischen Attribute ATT-1, ATT-2, ..., ATT-m zu empfangen und an das Modul 23 weiterzugeben. Dazu kann das Modul 24 dazu ausgebildet sein, eine grafische Benutzerschnittstelle GUI bereitzustellen, in der die inzidente medizinische Information I-MEDI bzw. die medizinische Information MEDI sowie die medizinischen Attribute ATT-1, ATT-2, ..., ATT-m und ggf. weitere Informationen/Datenobjekte darstellbar sind und über die eine Nutzereingabe NE eingebbar ist. Zusätzlich kann Modul 24 dazu ausgebildet sein, die medizinische Information MEDI zu archivieren (z.B. in der Speichereinheit 50 oder einer beliebigen anderen Datenbank) oder einem weiteren Modul bzw. einer weiteren Software zur Weiterverarbeitung bereitzustellen. Insbesondere kann auf diese Art eine Rückmeldung an eine ggf. bei der Ähnlichkeitsanalyse verwendete trainierte Funktion ALG-1 geschaffen werden, mit der die trainierte Funktion ALG-1 weiter optimiert werden kann.

Die vorgenommene Unterteilung der Recheneinheit 20 in Module 21-24 dient dabei lediglich der einfacheren Erklärung der Funktionsweise der Recheneinheit 20 und ist nicht beschränkend zu verstehen. Die Module 21-24 bzw. deren Funktionen können auch in einem Element zusammengefasst sein. Die Module 21-24 können dabei insbesondere auch als Computerprogrammprodukte oder Computerprogrammsegmente aufgefasst werden, welche bei der Ausführung in der Recheneinheit 20 ein oder mehrere der nachstehend beschriebenen Verfahrensschritte realisieren.

Die Recheneinheit 20 und der Prozessor 13 können zusammen die Steuerung 40 bilden. Es sei angemerkt, dass das gezeigte Layout der Steuerung 40, d.h. die geschilderte Aufteilung auf die Recheneinheit 20 und den Prozessor 13, ebenfalls nur beispielhaft zu verstehen ist. So kann die Recheneinheit 20 vollständig in dem Prozessor 13 integriert sein und umgekehrt. Insbesondere können die Verfahrensschritte durch ausführen eines entsprechenden Computerprogrammprodukts (z.B. einer auf der Nutzerschnittstelle installierten Software) vollständig auf dem Prozessor 13 der Nutzerschnittstelle 10 laufen, welche dann über die Schnittstelle 30 direkt z.B. mit der Speichereinheit 50 wechselwirkt. Mit anderen Worten wäre dann die Recheneinheit 20 identisch mit dem Prozessor 13.

Wie bereits erwähnt kann die Recheneinheit 20 gemäß einigen Ausführungsformen alternativ als Server-System, wie z.B. ein lokaler Server oder ein Cloud-Server, aufgefasst werden. Bei einer derartigen Ausgestaltung kann die Nutzerschnittstelle 10 als "Frontend" oder "Client" bezeichnet werden, während die Recheneinheit 20 dann als "Backend" aufgefasst werden kann. Eine Kommunikation zwischen der Nutzerschnittstelle 10 und der Recheneinheit 20 kann dann beispielsweise basierend auf einem https-Protokoll ausgeführt sein. Die Rechenleistung kann in solchen Systemen zwischen dem Client und dem Server aufgeteilt sein. In einem "Thin Client" System verfügt der Server über den Großteil der Rechenleistung, während der Client in einem "Thick Client" System mehr Rechenleistung bereitstellt. Ähnliches gilt für die Daten (hier: insbesondere der medizinische Datensatz PDS und die Vergleichsdatensätze VDS). Während im "Thin Client"-System die Daten meist auf dem Server verbleiben und nur die Ergebnisse an den Client übermittelt werden, werden dem Client im "Thick-Client"-System auch Daten übermittelt.

Gemäß weiteren Ausführungsformen kann die beschriebene Funktionalität auch als sog. Cloud-Service oder Web-Service bereitgestellt werden. Die entsprechend ausgebildete Recheneinheit ist dann als Cloud- oder Web-Plattform ausgebildet. In diese Plattform können dann die zu analysierenden Daten also die medizinischer Datensatz PDS hochgeladen über ein geeignetes Web-Interface hochgeladen werden.

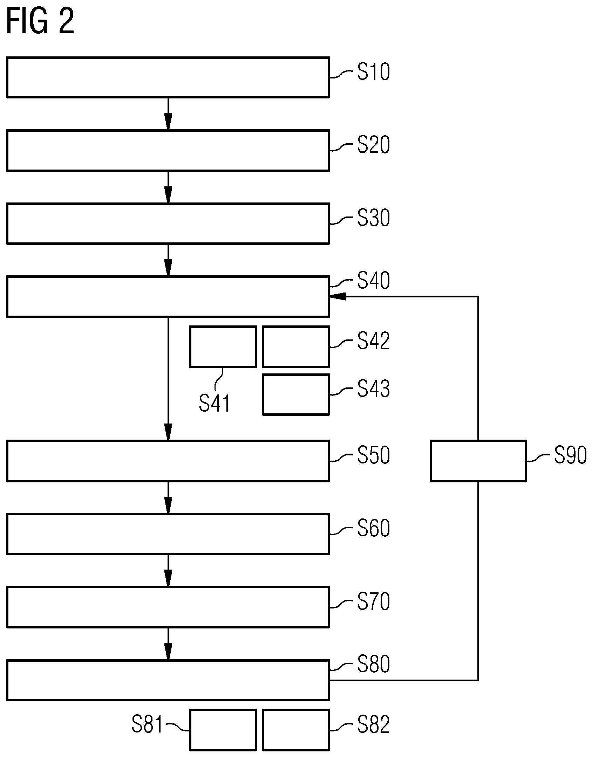

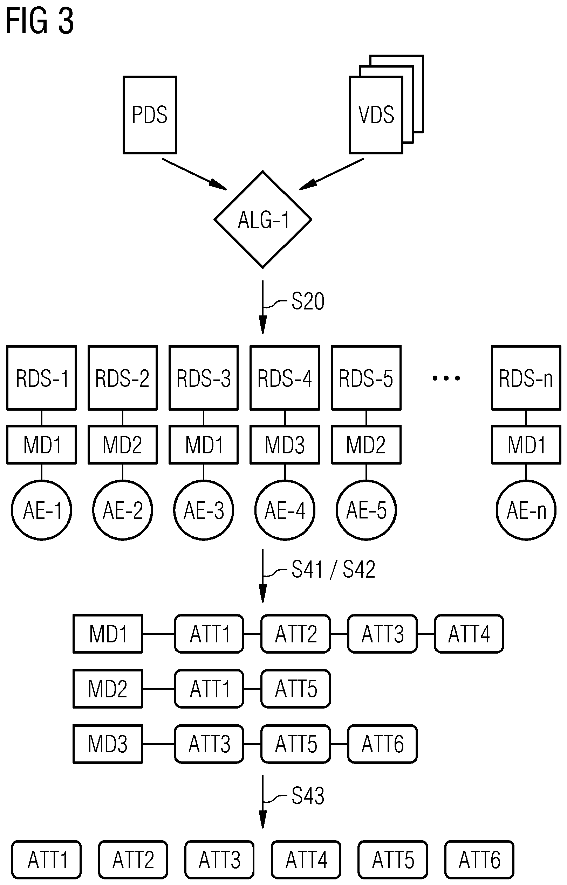

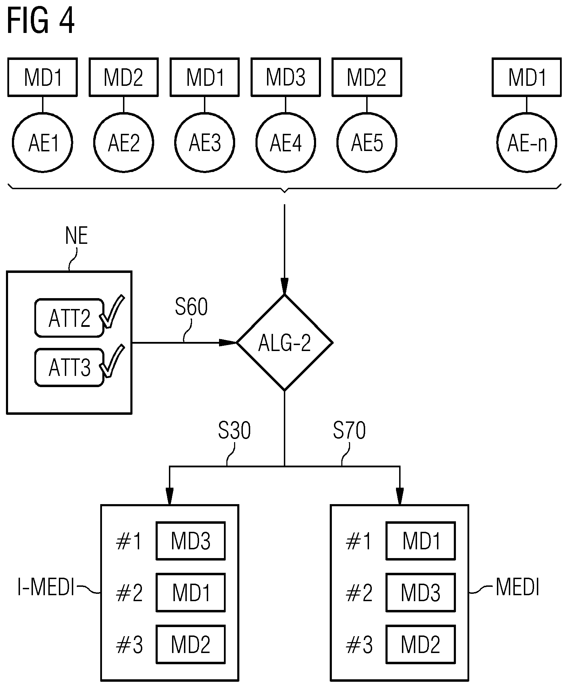

In **Figur 2** ist ein schematisches Ablaufdiagramm eines Verfahrens zur Bereitstellung einer medizinischen Information MEDI dargestellt. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. Außerdem können ein oder mehrere Schritte, insbesondere eine Sequenz von Schritten, und optional das gesamte Verfahren wiederholt ausgeführt werden. In **Figur 3** **und** 4 sind zugehörige Diagramme gezeigt, welche die mit dem in Figur 2 gezeigten Verfahren assoziierten Datenströme exemplarisch darstellen. Die in den Figuren 3 und 4 dargestellten Referenzdatensätze RDS-1, RDS-2, ..., RDS-n, medizinischen Attribute ATT-1, ATT-2, ..., ATT-m und medizinischen Befunde sind nur beispielhaft zu verstehen. Insbesondere werden sich je nach Fall unterschiedliche Anzahlen an Referenzdatensätze RDS-1, RDS-2, ..., RDS-n, medizinischen Attribute ATT-1, ATT-2, ..., ATT-m und medizinischen Befunde MD1, MD2, MD3 und unterschiedliche Zuordnungen ergeben.

Das in Figur 2 dargestellte Verfahren stellt in einigen Ausführungsformen darauf ab, basierend auf einer automatisierten Analyse der zu einem Patienten zur Verfügung stehenden Daten eine Auswahl möglicher Diagnosen/medizinscher Befunde anzubieten und durch eine fortgesetzte Mensch-Maschine-Interaktion einzugrenzen. Die zur Verfügung stehenden Daten sind einerseits durch den medizinischer Datensatz PDS des zu befundenen Patienten und andererseits durch Vergleichsdatensätze VDS gegeben. Jeder der Vergleichsdatensätze VDS betrifft, wie bereits ausgeführt, medizinische Datensätze zu (von dem zu befundenden Patienten verschiedenen) Vergleichspatienten. Die Vergleichsdatensätze VDS zeichnen sich dadurch aus, dass zu jedem Vergleichsdatensatz VDS wenigstens eine vorbekannte medizinische Diagnose MD1, MD2, MD3 bekannt ist, die für den zugehörigen Vergleichspatienten in der Vergangenheit getroffen wurde.

In einem ersten Schritt S10 wird der medizinische Datensatz PDS des zu befundenden Patienten bereitgestellt. Dies kann eine manuelle Auswahl des jeweiligen Falls durch einen Nutzer über die Nutzerschnittstelle 10 umfassen. Ferner kann dies ein Laden des medizinischen Datensatzes PDS von der Speichereinheit 50 oder einem anderen Datenspeicher umfassen, der beispielsweise Teil eines medizinischen Informationssystems sein kann. Zudem kann Schritt S10 ein Empfangen des medizinischen Datensatzes PDS durch die Recheneinheit 40 umfassen.

Gemäß einer Idee der vorliegenden Erfindung wird der medizinische Datensatz PDS von der Recheneinheit 40 automatisch mit den Vergleichsdatensätzen VDS verglichen, um unter den Vergleichsdatensätzen VDS Datensätze zu identifizieren, die eine gewisse Ähnlichkeit zu dem medizinischen Datensatz PDS aufweisen. Diese ähnlichen Datensätze werden auch Referenzdatensätze RDS-1, RDS-2, ..., RDS-n genannt. Unter der Annahme, dass ähnliche medizinische Datensätze ähnliche medizinische Befunde MD1, MD2, MD3 indizieren, kann aus den vorbekannten medizinischen Befunden MD1, MD2, MD3 der Referenzdatensätze RDS-1, RDS-2, ..., RDS-n auf mögliche medizinische Befunde MD1, MD2, MD3 für den medizinischer Datensatz PDS geschlossen werden.

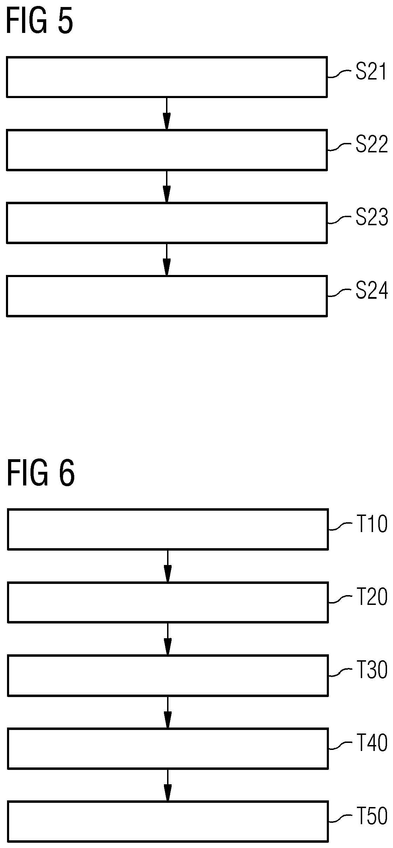

In Schritt S20 werden entsprechend aus den Vergleichsdatensätzen VDS ein oder mehrere Referenzdatensätze RDS-1, RDS-2, ..., RDS-n ermittelt, die eine bestimmte Ähnlichkeit zu dem medizinischen Datensatz PDS aufweisen. Schritt S20 beruht auf einer Ähnlichkeitsanalyse, bei der Ähnlichkeiten zwischen dem medizinischen Datensatz PDS und den Vergleichsdatensätzen VDS identifiziert bzw. quantifiziert werden. Die Ähnlichkeiten können dabei durch Ähnlichkeitsmaße AE-1, AE-2, ..., AE-n ausgedrückt werden. Insbesondere kann für wenigstens eine Teilmenge der Vergleichsdatensätze VDS jeweils ein Ähnlichkeitsmaß AE-1, AE-2, ..., AE-n ermittelt werden, das jeweils eine Ähnlichkeit des jeweiligen Vergleichsdatensatzes VDS mit dem medizinischen Datensatz PDS ausdrückt. Alle Vergleichsdatensätze VDS mit einem gewissen Ähnlichkeitsgrad zum medizinischer Datensatz PDS können als Referenzdatensätze RDS-1, RDS-2, ..., RDS-n identifiziert werden, der für die Befundung des medizinischen Datensatzes PDS potentiell relevant ist. Beispielsweise können all jenen Vergleichsdatensätze VDS als Referenzdatensätze RDS-1, RDS-2, ..., RDS-n identifiziert werden, deren Ähnlichkeitsmaß oberhalb einer vorgegebenen oder vorgebbaren Schwelle liegt. Alternativ können die Vergleichsdatensätze VDS mit den vergleichsweise höchsten Ähnlichkeitsmaßen als Referenzdatensätze RDS-1, RDS-2, ..., RDS-n identifiziert werden. Schritt S20 führt somit, wie in Figur 4 gezeigt, zu einem oder mehreren Referenzdatensätzen RDS-1, RDS-2, ..., RDS-n. Zu jedem Referenzdatensatz RDS-1, RDS-2, ..., RDS-n gehört ein entsprechendes Ähnlichkeitsmaß AE-1, AE-2, ..., AE-n. Gemäß Ausführungsformen der Erfindung wird die Ähnlichkeitsanalyse in Schritt S20 von dem ersten Datenverarbeitungsalgorithmus ALG-1 durchgeführt. Eine eingehendere Beschreibung der Ermittlung der Ähnlichkeitsmaße wird zudem in Bezug auf Figur 5 gegeben.

Aufgrund der Ähnlichkeitsanalyse kommen die mit den Referenzdatensätzen RDS-1, RDS-2, ..., RDS-n assoziierten medinischen Befunden MD1, MD2, MD3 auch für den zu befundenden Patienten in Frage. Wie in Figur 3 gezeigt, ist jeder Referenzdatensatz RDS-1, RDS-2, ..., RDS-n mit einem medizinischen Befund MD1, MD2, MD3 assoziiert. Dabei können verschiedene Referenzdatensätze RDS-1, RDS-2, ..., RDS-n mit identischen medizinischen Befund MD1, MD2, MD3 assoziiert sein. Gemäß einigen Beispielen kann ein Referenzdatensatz RDS-1, RDS-2, ..., RDS-n auch mit mehr als einem medizinischen Befund assoziiert sein (in Figur 3 nicht dargestellt). Dabei liefern die Ähnlichkeitsmaße AE-1, AE-2, ..., AE-n einen Anhaltspunkt, wie wahrscheinlich ein medizinischer Befund MD1, MD2, MD3 aus einem Referenzdatensätze RDS-1, RDS-2, ..., RDS-n auch für den medizinischen Datensatz PDS zutreffend ist. Kann ein medizinsicher Befund MD1, MD2, MD3 mit durchweg hohen Ähnlichkeitsmaßen AE-1, AE-2, ..., AE-n in Verbindung gebracht werden, ist er relevanter als ein medizinischer Befund MD1, MD2, MD3 mit geringeren Ähnlichkeitsmaßen AE-1, AE-2, ..., AE-n.

In Schritt S30 wird dieser Zusammenhang verwendet, um eine erste oder inzidente medizinische Information I-MEDI abzuleiten, die dem Nutzer Informationen über mögliche medizinische Befunde MD1, MD2, MD3 für den zu befundenden Patienten und, optional, deren mögliche Relevanz bzw. Einschlägigkeit bereitstellt. Die Relevanz bzw. Einschlägigkeit kann wie oben ausgeführt aus den Ähnlichkeitsmaßen AE-1, AE-2, ..., AE-n abgeleitet werden. Dazu können in Schritt S30 beispielsweise die Ähnlichkeitsmaße AE-1, AE-2, ..., AE-n ausgewertet werden, um z.B. einen Konfidenzwert des jeweiligen medizinischen Befundes MD1, MD2, MD3 zu erhalten. Dieser Konfidenzwert kann als "Punktzahl" (engl.: Score) für den jeweiligen medizinischen Befund MD1, MD2, MD3 implementiert sein. Ein Konfidenzwert kann z.B. durch die Berechnung mittlerer Ähnlichkeitsmaße AE-1, AE-2, ..., AE-n oder einer ggf. gewichteten Summe der Ähnlichkeitsmaße AE-1, AE-2, ..., AE-n implementiert sein. Die inzidente medizinische Information I-MEDI kann auf dieser Grundlage beispielsweise eine Rangfolge der medizinischen Befunde MD1, MD2, MD3 umfassen. Die Rangfolge würde dann angeben, wie zutreffend ein jeweiliger medizinischer Befund MD1, MD2, MD3 im Vergleich zu anderen medizinischen Befunden MD1, MD2, MD3 basierend auf der Ähnlichkeitsanalyse ist. Im in Figur 4 gezeigten Beispiel ist in der inzidenten medizinischen Information I-MEDI der medizinische Befund MD3 an erster Stelle der Rangfolge, und somit auf Grundlage der Ähnlichkeitsanalyse (der Ähnlichkeitsmaße AE-1, AE-2, ..., AE-n) am relevantesten für den zu befundenden Patienten einzuschätzen. An zweiter Stelle kommt der medizinische Befund MD1 und an dritter Stelle der medizinische Befund MD2. Alternativ oder zusätzlich kann die initiale medizinische Information I-MEDI auch die o.g. Konfidenzwerte enthalten. Gemäß Ausführungsformen der Erfindung kann die initiale medizinische Information I-MEDI durch eine Anwendung des zweiten Datenverarbeitungsalgorithmus ALG-2 bereitgestellt werden.

In einem optionalen Teilschritt S31 kann die inzidente medizinische Information I-MEDI dem Nutzer über die Nutzerschnittstelle 10 angezeigt werden. Bevorzugt geschieht dies in einer Darstellungsform, die dazu geeignet ist, dass der Nutzer die Relevanz eines jeweiligen medizinischen Befunds MD1, MD2, MD3 im Vergleich zu den anderen medizinischen Befunden MD1, MD2, MD3 wahrnehmen kann. Wie nachfolgend in Bezugnahme auf Figuren 6 bis 8 noch näher beschrieben wird, kann hier beispielsweise mit der Anzeige von Listen und/oder Zahlenwerten und/oder Markierungen gearbeitet werden.

Die durch die Ähnlichkeitsanalyse gefundenen medizinischen Befunde MD1, MD2, MD3 und deren relative Relevanzeinschätzung (d.h. die initiale medizinische Information I-MEDI) basiert auf den zur Verfügung stehenden Daten. Sie wird automatisch erzeugt und enthält noch keinen Input des Nutzers. Ausführungsformen der vorliegenden Erfindung sehen vor, die initiale medizinische Information I-MEDI durch eine fortgesetzte und zielgerichtete Mensch-Maschine-Interaktion zu präzisieren. Dabei ist vorgesehen, dem Nutzer Tags oder medizinische Attribute ATT-1, ATT-2, ..., ATT-m anzubieten. Der Nutzer kann dann durch eine Nutzereingabe NE entscheiden, ob und ggf. in welchem Maße die medizinischen Attribute ATT-1, ATT-2, ..., ATT-m für den zu befundenden Patienten zutreffen. Diese Beurteilung ist regelmäßig deutlich einfacher als die Einschätzung ob ein kompletter medizinischer Befund MD1, MD2, MD3 zutrifft oder nicht. Die Präzisierung der inzidenten medizinischen Information I-MEDI wird folglich in für den Nutzer einfacher zu handhabende Teilprobleme zerlegt. Die medizinischen Attribute ATT-1, ATT-2, ..., ATT-m beziehen sich auf übergeordnete Eigenschaften oder Kennzeichen des jeweiligen medizinischen Befunds MD1, MD2, MD3. Die medizinischen Attribute ATT-1, ATT-2, ..., ATT-m können verschiedene Kategorien in Zusammenhang mit medizinischen Befunden betreffen. So können medizinische Attribute ATT-1, ATT-2, ..., ATT-m z.B. Angaben zu Symptomen, klinischen Anzeichen oder Gegenanzeichen, zu Patienteninformationen, Risikofaktoren, Differentialdiagnosen usw. umfassen. Im Hinblick auf den klinischen Verlauf kann ein medizinisches Attribut beispielsweise angeben, ob eine akute, sub-akute oder chronischen Erkrankung vorliegt. Am Beispiel einer Lungenerkrankung kann ein Attribut beispielsweise angeben, ob ein Krankheitsbild einseitig oder beidseitig vorliegt. Ein Attribut ATT-1, ATT-2, ..., ATT-m eines medizinischen Befunds MD1, MD2, MD3 kann auch sein, wie häufig oder selten das zugrundeliegende Krankheitsbild vorkommt oder ob eine Infektionskrankheit vorliegt. Wie bereits erwähnt können die Attribute ATT-1, ATT-2, ..., ATT-m insbesondere als semantische Kennzeichen vorliegen. Wie in den Figuren 3 und 4 dargestellt, kann ein medizinscher Befund mehrere verschiedene medizinische Attribute ATT-1, ATT-2, ..., ATT-m aufweisen. Außerdem kann ein medizinisches Attribute ATT-1, ATT-2, ..., ATT-m für mehrere verschiedene medizinische Befunde MD1, MD2, MD3 zutreffen. Darüber hinaus können sich verschiedene medizinischen Attribute ATT-1, ATT-2, ..., ATT-m gegenseitig ausschließen.

In Schritt S40 werden die für die weitere Eingrenzung der medizinischen Befunde MD1, MD2, MD3 und Präzisierung der inzidenten medizinischen Information I-MEDI relevanten medizinischen Attribute ATT-1, ATT-2, ..., ATT-m ermittelt. Relevante medizinischen Attribute ATT-1, ATT-2, ..., ATT-m sind dabei diejenigen medizinischen Attribute ATT-1, ATT-2, ..., ATT-m, die für die durch die Ähnlichkeitsanalyse ermittelten medizinischen Befunde MD1, MD2, MD3 einschlägig sind. In einem optionalen Teilschritt S41 kann dazu eine Zuordnung oder Zuordnungsvorschrift bereitgestellt werden, die medizinischen Befunden MD1, MD2, MD3 ein oder mehrere medizinische Attribute ATT-1, ATT-2, ..., ATT-m zuordnet. Die Zuordnung kann dabei insbesondere vorbekannt sein und beispielsweise basierend auf Annotationen durch einen oder mehrere Experten und/oder einer Auswertung von Lehrbuchwissen oder medizinischen Richtlinien erstellt werden. In einem weiteren optionalen Teilschritt S42 werden den medizinischen Befunden MD1, MD2, MD3 jeweils diese kennzeichnenden medizinischen Attribute ATT-1, ATT-2, ..., ATT-m zugeordnet. Als Alternative hierzu kann auch ein elektronischer (trainierter) Klassifikator verwendet werden, der jedem medizinischen Befund MD1, MD2, MD3 ein oder mehrere medizinische Attribute ATT-1, ATT-2, ..., ATT-m zuordnet. Als weitere Alternative können ein oder mehrere medizinischen Attribute ATT-1, ATT-2, ..., ATT-m in den zugehörigen Referenzdatensätze RDS-1, RDS-2, ..., RDS-n hinterlegt sein und durch Auswertung der Referenzdatensätze RDS-1, RDS-2, ..., RDS-n aufgefunden werden. Wie in Figur 3 gezeigt, werden die medizinischen Befunde MD1, MD2, MD3 mit denen ihnen jeweils zugeordneten medizinischen Attributen ATT-1, ATT-2, ..., ATT-m erhalten. Optional können diese medizinischen Attribute ATT-1, ATT-2, ..., ATT-m in Schritt S30 oder S90 zusammen mit den jeweiligen medizinischen Befunden dargestellt werden (vgl. auch Figuren 6 bis 8).

Wie in Figur 3 ferner gezeigt ist, wird aus den medizinischen Attributen ATT-1, ATT-2, ..., ATT-m der medizinischen Befunde MD1, MD2, MD3 ein Satz medizinischer Attribute ATT-1, ATT-2, ..., ATT-m gebildet, der dem Nutzer zur Beurteilung angezeigt werden soll (Teilschritt S43). Dabei können z.B. Doppelnennungen von medizinischen Attribute ATT-1, ATT-2, ..., ATT-m eliminiert werden. Optional können dabei auch medizinische Attribute ATT-1, ATT-2, ..., ATT-m herausgefiltert werden. Das kann insbesondere dann sinnvoll sein, wenn ein medizinisches Attribut nur in Zusammenhang mit einem medizinischen Befund auftritt, der ein geringes Ähnlichkeitsmaß AE-1, AE-2, ..., AE-n aufweist, weil das bedeuten kann, dass ein solches medizinisches Attribut ATT-1, ATT-2, ..., ATT-m nur eine geringe diskriminierende Wirkung zur weiteren Eingrenzung eines medizinischen Befunds MD1, MD2, MD3 entfalten wird.

In einem Schritt S50 werden die in Schritt S40 ermittelten medizinischen Attribute ATT-1, ATT-2, ..., ATT-m dem Nutzer über die Nutzerschnittstelle 10 angezeigt. Der Zweck dieser Anzeige ist, dem Nutzer die ermittelten medizinischen Attribute ATT-1, ATT-2, ..., ATT-m für eine Verifikation und/oder Bewertung bereitzustellen. Optional kann dabei eine Darstellungsform gewählt werden, in der die medizinischen Attribute ATT-1, ATT-2, ..., ATT-m nach Kategorien gruppiert und/oder nach ihrer diskriminierenden Wirkung geordnet sind. Die diskriminierende Wirkung kann dabei beispielsweise anhand der Ähnlichkeitsmaße AE-1, AE-2, ..., AE-n und/oder Simulation eines zu einem oder mehreren medizinischen Befunden MD1, MD2, MD3 führenden Entscheidungsbaums bestimmt werden. Die Verifikation bzw. Bewertung der medizinischen Attribute ATT-1, ATT-2, ..., ATT-m durch den Nutzer erfolgt dabei durch eine Eingabe einer entsprechenden Nutzereingabe NE. Eine Nutzereingabe NE kann auf eine Bestätigung und/oder ein Verwerfen bzw. Ausschließen einzelner medizinischer Attribute ATT-1, ATT-2, ..., ATT-m für den zu befundenden Fall gerichtet sein. Neben einer solchen qualitativen Angabe kann die Nutzereingabe NE zur weiteren Differenzierung eine quantitative Angabe umfassen, die ein Maß dafür enthält, wie zutreffend einzelne medizinische Attribute ATT-1, ATT-2, ..., ATT-m für den zu befundenden Fall sind (beispielsweise kann ein solches Maß ausgewählt sein aus einem Bereich von 100% zutreffend bis 0% zutreffend). Bevorzugt erfolgt die Anzeige in Schritt S50 über eine grafische Benutzerschnittstelle GUI, über die auch die Nutzereingabe NE eingegeben werden kann.

Empfangen wird die Nutzereingabe NE in Schritt S60. Die Nutzereingabe NE kann durch den Nutzer beispielsweise durch Betätigen ein oder mehrerer Schaltflächen in einer grafischen Benutzerschnittstelle GUI sowie durch Text- oder Spracheingabe oder andere Arten erfolgen, mit denen Daten in das System 1 eingegeben werden können.

In Schritt S70 wird die Nutzereingabe NE ausgewertet. Insbesondere wird in Schritt S70 eine medizinische Information MEDI erstellt, in der die Nutzereingabe NE berücksichtigt ist. Dabei wird ausgenutzt, dass eine Verifikation bzw. Bewertung eines medizinischen Attributs ATT-1, ATT-2, ..., ATT-m durch den Nutzer unmittelbare Auswirkungen darauf hat, inwieweit der zugehörige medizinische Befund MD1, MD2, MD3 auf den vorliegenden Fall zutrifft. Enthält die Nutzereingabe NE beispielsweise eine Auswahl eines bestimmten medizinischen Attributs ATT-1, ATT-2, ..., ATT-m für den vorliegenden Fall (im in Figur 4 gezeigten Beispiel ATT2 und ATT3), ist dies als Indiz dafür zu werten, dass die mit diesem medizinischen Attribut ATT-1, ATT-2, ..., ATT-m assoziierten medizinischen Befunde MD1, MD2, MD3 für den zu befundenden Fall relevanter sind, als solche die das medizinische Attribut ATT-1, ATT-2, ..., ATT-m nicht zeigen (oder sogar ausschließen). Je mehr durch den Nutzer ausgewählte medizinischen Attribute ATT-1, ATT-2, ..., ATT-m für einen medizinischen Befund MD1, MD2, MD3 zutreffen, desto größer ist dessen mögliche Relevanz für den zu befundenden Fall. Umgekehrt kann der Ausschluss eines medizinischen Attributs ATT-1, ATT-2, ..., ATT-m zum Ausschluss der medizinischen Befunde MD1, MD2, MD3 führen, die diese medizinische Attribut ATT-1, ATT-2, ..., ATT-m erfordern. Durch Auswertung der Nutzereingabe NE kann mit anderen Worten für jeden der durch die Ähnlichkeitsanalyse gefundenen medizinischen Befunde MD1, MD2, MD3 ein Konfidenzwert (etwa in Form einer Punktzahl) gefunden werden, der die relative Relevanz der medizinischen Befunde MD1, MD2, MD3 für den zu befundenden Patienten angibt. Auf dieser Grundlage kann, ähnlich wie in Schritt S30 beschrieben, eine medizinische Information MEDI ermittelt werden, die Informationen über mögliche medizinische Befunde MD1, MD2, MD3 für den zu befundenden Patienten und, optional, deren mögliche Relevanz bzw. Einschlägig-keit bereitstellt. Die medizinische Information MEDI kann dabei lediglich auf den durch die Ähnlichkeitsanalyse ausgewählten medizinischen Befunden MD1, MD2, MD3 und der Nutzereingabe NE basieren. Zusätzlich kann die medizinische Information MEDI die Ähnlichkeitsmaße AE-1, AE-2, ..., AE-n berücksichtigen und somit die Ähnlichkeitsanalyse und die Nutzereingabe NE zusammenführen. Die medizinische Information MEDI kann dann als eine basierend auf der Nutzereingabe NE angepasste inzidente medizinische Information I-MEDI erzeugt werden. Gemäß Ausführungsformen der Erfindung kann die medizinische Information MEDI durch eine Anwendung des zweiten Datenverarbeitungsalgorithmus ALG-2 bereitgestellt werden.

Der folgende Schritt S80 ist auf die Bereitstellung der medizinischen Information MEDI gerichtet. Die Bereitstellung kann eine Anzeige der medizinischen Information MEDI etwa über die Nutzerschnittstelle 10 umfassen (Teilschritt S81). Alternativ oder zusätzlich kann die medizinische Information MEDI weiterführenden Bearbeitungsprozessen bereitgestellt werden (Teilschritt S82). Solche weiterführende Verarbeitungsprozesse können beispielsweise die (teil-)automatisierte Erstellung eines medizinischen Berichts und dabei insbesondere das automatisierte Befüllen eines Berichtsvordrucks umfassen. Ferner können weiterführende Verarbeitungsprozesse das Archivieren der medizinischen Information MEDI vorzugsweise in Zuordnung zu dem medizinischen Datensatz PDS umfassen. Darüber hinaus kann als weiterer Verarbeitungsprozess ein Bereitstellen der medizinischen Information MEDI an den ersten Datenverarbeitungsalgorithmus ALG-1 umfassen. Dem liegt der Gedanke zu Grunde, dass sich eine Eingrenzung der medizinischen Befunde MD1, MD2, MD3 durch den Nutzer implizit eine Bewertung z.B. durch den ersten Datenverarbeitungsalgorithmus ALG-1 gefundenen Ähnlichkeitsmaße AE-1, AE-2, ..., AE-n enthält. Damit kann die medizinische Information MEDI zu einer Verbesserung des ersten Datenverarbeitungsalgorithmus ALG-1 genutzt werden. Bei Teilschritt S82 ist es bevorzugt, dass der Nutzer der Bereitstellung (z.B. mit einer Eingabe in die entsprechend ausgebildete grafische Benutzerschnittstelle GUI) zustimmen bzw. aktiv einleiten muss.

Schritt S90 ist ein Wiederholungsschritt. Wenn der Nutzer z.B. auf die Anzeige in Schritt S81 eine weitere Nutzereingabe NE vornimmt, sorgt Schritt S90 dafür, dass die medizinische Information MEDI an die weitere Nutzereingabe NE angepasst (Schritte S60 und S70) und die Bereitstellung entsprechend aktualisiert wird (Schritt S80). Die weitere Nutzereingabe NE kann beispielsweise die Aus- oder Abwahl weiterer medizinischer Attribute ATT-1, ATT-2, ..., ATT-m umfassen.

**Figur 5** zeigt eine beispielhafte Ausführungsform für Einzelschritte zur Ermittlung von Referenzdatensätzen RDS-1, RDS-2, ..., RDS-n. Die Einzelschritte können insbesondere innerhalb von Schritt S20 ausgeführt werden. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. Außerdem können ein oder mehrere Schritte, insbesondere eine Sequenz von Schritten bis hin zu allen Schritten wiederholt ausgeführt werden.

In einem ersten Schritt S21 wird aus dem medizinischen Datensatz PDS ein Datendeskriptor erzeugt. Der Datendeskriptor kann wesentliche Merkmale des medizinischen Datensatzes PDS in Form eines Merkmalsvektors umfassen. Da der medizinische Datensatz PDS im Allgemeinen Bilddaten und Nicht-Bilddaten aufweist, kann auch der Datendeskriptor auf Bild-Merkmalen und Nicht-Bild-Merkmalen basieren. Bild-Merkmale können unter Verwendung von Bildverarbeitungsverfahren extrahiert werden. Diese können die Identifizierung, Analyse und/oder Vermessung von Objekten, lokalen und/oder globalen Strukturen, Mustern oder Texturen umfassen, die in den Bilddaten des medizinischen Datensatzes PDS enthalten sind. Die Merkmale können ferner anatomische Merkmale und/oder Strukturen, wie etwa die Gegenwart eine anatomische Landmarke oder die Größe, Textur oder Dichte eines identifizierten Organs umfassen. Ferner können die Merkmale Parameter umfassen, die Bildwerte der im medizinischen Datensatz PDS enthaltenen Bilddaten charakterisieren. Dies können z.B. Parameter sein, die eine Farbe, eine Graustufe, einen Kontrast oder Gradienten dieser Größen beschreiben. Die aus den Nicht-Bilddaten extrahierten Merkmale können z.B. Metadaten zu den Bilddaten des medizinischen Datensatzes PDS umfassen. Weiterhin können sich die Merkmale auf weitere Kontextdaten des zu befundenden Patienten beziehen. Diese Merkmale können sich z.B. auf demografische Angaben zum Patienten, einer oder mehrere Vorerkrankungen, Risikofaktoren, bereits vorhandene Diagnosen und Befunde, Laborwerte, Vitalwerte, usw. beziehen. Der Datendeskriptor weist bevorzugt eine Vielzahl an Merkmalen auf, die in Summe den medizinischer Datensatz PDS charakterisieren. In einigen Ausführungsformen wird der Schritt S21 unter Verwendung des ersten Datenverarbeitungsalgorithmus ALG-1 vollzogen.

In Schritt S22 von Figur 5 wird der Datendeskriptor verwendet, um die Speichereinheit 50 abzufragen. Hierfür kann der Datendeskriptor mit den korrespondierenden Datendeskriptoren der Vergleichsdatensätze VDS verglichen werden. Die korrespondieren Datendeskriptoren können analog zu dem Datendeskriptor des medizinischen Datensatzes PDS erzeugt werden und insbesondere die gleiche Struktur wie dieser aufweisen. Gemäß einigen Ausführungsformen wurden die korrespondierenden Datendeskriptoren bereits vor der Abfrage in Schritt S22 erzeugt und zusammen mit den Vergleichsdatensätzen VDS in der Speichereinheit 50 gespeichert.

Im folgenden Schritt S23 wird eine Ähnlichkeitsmetrik ausgewertet, mit welcher eine Ähnlichkeit zwischen dem Datendeskriptor des medizinischen Datensatzes PDS und den korrespondieren Datendeskriptoren der Vergleichsdatensätze VDS quantifiziert werden kann. Dabei können alle oder nur ein Teil der in der Speichereinheit enthaltenen Vergleichsdatensätze VDS berücksichtigt werden. Mit anderen Worten können so in Schritt S23 für jeden berücksichtigten Vergleichsdatensatz VDS ein Ähnlichkeitsmaß AE-1, AE-2, ..., AE-n erhalten werden. Gemäß einigen Ausführungsformen kann die Ähnlichkeitsmetrik ein Abstand im Vektorraum zwischen dem Merkmalsvektoren des medizinischer Datensatz PDS und der Vergleichsdatensätze VDS sein. Beispielsweise kann der Abstand als euklidischer Abstand gegeben sein. Gemäß weiteren Beispielen kann die Ähnlichkeitsmetrik als Cosinus-Ähnlichkeit zwischen den Datendeskriptoren definiert sein. Gemäß anderen Beispielen kann die Ähnlichkeit einzelner Merkmale individuelle gewichtet werden.

In Schritt S24 werden die in Schritt S23 ermittelten Ähnlichkeitsmaße AE-1, AE-2, ..., AE-n dazu verwendet, aus den Vergleichsdatensätzen VDS diejenigen Datensätze auszuwählen, die eine gewisse Ähnlichkeit zu dem medizinischer Datensatz PDS aufweisen. Die ausgewählten Datensätze bilden die in den Schritten S30 ff. verwendeten Referenzdatensätze RDS-1, RDS-2, ..., RDS-n. Gemäß beispielhaften Ausführungsformen können alle Vergleichsdatensätze VDS ausgewählt deren Ähnlichkeitsmaß oberhalb einer vorbestimmten Schwelle ist.

Das Ergebnis der Schritte S21-S24 sind also ein oder mehrere Referenzdatensätze RDS-1, RDS-2, ..., RDS-n mit zugehörigen Ähnlichkeitsmaße AE-1, AE-2, ..., AE-n, die jeweils eine Ähnlichkeit des jeweiligen Referenzdatensatzes RDS-1, RDS-2, ..., RDS-n zum medizinischen Datensatz PDS angeben. Einer oder mehrere - und insbesondere alle der Schritte S21-S24 - können von dem (entsprechend ausgebildeten) ersten Datenverarbeitungsalgorithmus ALG-1 ausgeführt werden. Dazu kann der erste Datenverarbeitungsalgorithmus ALG-1 ein oder mehrere trainierte Funktionen aufweisen. Je nach Auslegung kann eine trainierte Funktion mehr oder weniger Schritte S21-S24 ausführen. Insbesondere kann das Extrahieren der Merkmalsignaturen mit Hilfe einer trainierten Funktion erfolgen, aus denen dann die Ähnlichkeitsmaße AE-1, AE-2, ..., AE-n errechnet werden. Alternativ kann eine trainierte Funktion auch derart ausgelegt sein, die Ähnlichkeitsmaße AE-1, AE-2, ..., AE-n unmittelbar anzugeben. Neben trainierten Funktionen können selbstverständlich auch klassische, nicht auf künstlicher Intelligenz beruhende Funktionen verwendet werden. Ein Beispiel hierfür wären sog. Textur-Klassifikationsalgorithmen.

Die für den ersten Datenverarbeitungsalgorithmus ALG-1 verwendeten trainierten Funktionen können gemäß einigen Ausführungsformen ein neuronales Netz aufweisen. Neuronale Netzwerk können eine Vielzahl von aufeinanderfolgenden Schichten aufweisen. Jede Schicht umfasst mindestens einen, vorzugsweise mehrere Knoten. Im Wesentlichen kann jeder Knoten eine mathematische Operation ausführen, die einen oder mehrere Eingabewerte einem Ausgabewert zuordnet. Die Knoten jeder Schicht können mit allen oder nur einer Teilmenge von Knoten einer vorherigen und/oder nachfolgenden Schicht verbunden sein. Zwei Knoten sind "verbunden", wenn ihre Ein- und/oder Ausgänge verbunden sind. Die Kanten oder Verbindungen sind mit einem Parameter assoziiert, der häufig als "Gewicht" oder "Kantengewicht" bezeichnet wird. Eingabewerte für die Knoten der jeweils ersten Schicht können beispielsweise der medizinischer Datensatz PDS und die Vergleichsdatensätze VDS sein. Die jeweils letzte Schicht wird oft als Ausgabeschicht bezeichnet. Ausgabewerte der Knoten der Ausgabeschicht können je nach Auslegung beispielsweise Merkmalssignaturen (die dann noch in Ähnlichkeitsmaße AE-1, AE-2, ..., AE-n überführt werden müssten) oder gleich Ähnlichkeitsmaße AE-1, AE-2, ..., AE-n sein. Zwischen der Eingabeschicht und der Ausgabeschicht befindet sich eine Anzahl von verborgenen Schichten (ein englischer Fachbegriff ist "hidden layers").

Gemäß einigen Ausführungsformen können die trainierten Funktionen insbesondere ein faltendes neuronales Netzwerk (ein englischer Fachbegriff hierfür ist "convolutional neural network" oder kurz CNN) oder ein tiefes faltendes neuronales Netzwerk (ein englischer Fachbegriff ist "deep convolutional neural network") aufweisen. Solche trainierten Funktionen weisen dann ein oder mehrere Faltungsschichten (ein englischer Fachbegriff ist "convolutional layer") und, optional, ein oder mehrere Entfaltungsschichten (ein englischer Fachbegriff ist "deconvolutional layer") auf. Ferner kann die trainierte Funktion Sammelschichten (ein englischer Fachbegriff ist "pooling layer") und Up-Sampling-Schichten sowie vollständig verbundene Schichten (ein englischer Fachbegriff ist "fully connected layer") aufweisen. Faltungsschichten falten die Eingabe und geben ihr Ergebnis an die nächste Ebene weiter, indem ein Bildfilter über die Eingabe bewegt wird. Faltungsschichten können sich insbesondere dann als vorteilhaft erweisen, wenn, wie in einigen Ausführungsformen, nach ähnlichen Bildbereichen gesucht werden soll. Die Sammelschichten reduzieren die Dimensionen der Daten, indem die Ausgaben von Knotengruppen einer Schicht auf einen einzelnen Knoten in der nächsten Schicht zusammengefasst werden. Up-Sampling-Schichten und Entfaltungsschichten kehren die Aktionen der Faltungsschichten und der Sammelschichten um. Vollständig verbundene Schichten verbinden jeden Knoten vorhergehender Schichten mit Knoten nachfolgender Schichten, so dass im Wesentlichen jeder Knoten eine "Stimme" erhält.

Auch der zweite Datenverarbeitungsalgorithmus ALG-2 kann ein oder mehrere trainierte Funktionen umfassen. Hierfür können gemäß einigen Ausführungsformen ebenfalls neuronale Netzwerke beschrieben werden, die dazu ausgelegt sind, basierend auf den zur Verfügung stehenden Eingangsdaten (d.h. den Ähnlichkeitsmaße AE-1, AE-2, ..., AE-n, den medizinischen Befunden MD1, MD2, MD3 und/oder den Nutzereingaben NE) eine medizinische Indikation I-MEDI/MEDI auszugeben. Daneben kann der zweite Datenverarbeitungsalgorithmus ALG-2 ein oder mehrere trainierte Klassifikatoren, wie etwa ein k-nächste-Nachbarn-Netzwerk oder Stützvektormaschinen (ein englischer Fachbegriff hierfür ist "Support Vector Machine", oder kurz SVM) aufweisen. Daneben kann der zweite Datenverarbeitungsalgorithmus ALG-2 selbstverständlich auch auf einer klassischen Funktion beruhen, die keine künstliche Intelligenz verwendet.

Eine trainierte Funktion lernt durch Anpassen von Parametern, welche die Abbildung von Eingangs- auf Ausgangsdaten bestimmen. Bei neuronalen Netzwerken sind dies z.B. die Gewichte oder Gewichtungsparameter (z.B. der Kantengewichte) einzelner Schichten und Knoten. Eine trainierte Funktion kann beispielsweise durch Verfahren des überwachten Lernens trainiert werden (ein englischer Fachbegriff hierfür ist "supervised learning"). Hier kann beispielsweise das Verfahren der Rückpropagation verwendet werden (ein englischer Fachbegriff hierfür ist "back propagation"). Während des Trainings wird die trainierte Funktion auf Trainingseingangsdaten angewendet, um entsprechende Ausgabewerte zu erzeugen, deren Zielwerte in Form von Trainingsausgangsdaten bekannt sind. Die Differenz zwischen den Ausgabewerten und den Trainingsausgangsdaten kann verwendet werden, um eine Kosten- oder Verlustfunktional als Maß dafür einzuführen, wie gut oder schlecht die trainierte Funktion die ihr gestellte Aufgabe erfüllt. Ziel des Trainings ist es, ein (lokales) Minimum des Kostenfunktionals zu finden, indem die Parameter (z.B. die Kantengewichte) der trainierten Funktion iterativ angepasst werden. Die trainierte Funktion wird dadurch schließlich in die Lage versetzt, akzeptable Ergebnisse über eine (ausreichend) große Kohorte von Trainingseingangsdaten zu liefern. Dieses Optimierungsproblem kann unter Verwendung eines stochastischen Gradientenverfahrens (ein englischer Fachbegriff hierfür ist "stochastic gradient descent") oder anderer auf dem Fachgebiet bekannte Ansätze durchgeführt werden.

Für den ersten Datenverarbeitungsalgorithmus ALG-1 würden, falls dieser eine trainierte Funktion aufweist, Trainingsdatensätze jeweils medizinische Trainings-Datensätze und Trainings-Vergleichsdatensätze sowie, je nach Konfiguration des ersten Datenverarbeitungsalgorithmus ALG-1, zugehörige verifizierte Merkmalssignaturen, verifizierte Referenzdatensätze und/oder verifizierte aufweisen. Die verifizierten Referenzdatensätze könnten dabei auf einer Annotation eines Nutzers beruhen, die dieser basierend auf einer Analyse bzw. Befundung des medizinischen Trainings-Datensatzes vorgenommen hat. Gleiches gilt für die verifizierten Ähnlichkeitsmaße.

Ein Training des ersten Datenverarbeitungsalgorithmus ALG-1 gemäß einigen Ausführungsformen der Erfindung könnte dann somit ein Anwenden des ersten Datenverarbeitungsalgorithmus ALG-1 auf die medizinischen Trainings-Datensätze bzw. Trainings-VDS zur Erzeugung von Ausgabewerten sowie ein Vergleichen der Ausgabewerte mit den verifizierte Merkmalssignaturen, verifizierte Referenzdatensätzen und/oder verifizierten Ähnlichkeitsmaßen umfassen. Basierend auf dem Vergleich können dann ein oder mehrere Parameter des ersten Datenverarbeitungsalgorithmus ALG-1 angepasst werden.

Ferner kann vorgesehen sein, den ersten Datenverarbeitungsalgorithmus ALG-1 durch Feedback der medizinischen Information MEDI fortwährend weiter zu trainieren. Ein entsprechendes Verfahren ist in **Figur 6** gezeigt. Die Reihenfolge der Verfahrensschritte ist weder durch die dargestellte Abfolge noch durch die gewählte Nummerierung beschränkt. So kann die Reihenfolge der Schritte ggf. vertauscht und einzelne Schritte können weggelassen werden. Außerdem können ein oder mehrere Schritte, insbesondere eine Sequenz von Schritten bis hin zu allen Schritten wiederholt ausgeführt werden.

Schritt T10 ist ein Bereitstellen eines medizinischen Datensatzes PDS und mehrerer Vergleichsdatensätze VDS gerichtet. Der medizinische Datensatz PDS bzw. die Vergleichsdatensätze VDS können insbesondere die oben beschriebene Form aufweisen.

In Schritt T20 werden durch den ersten Datenverarbeitungsalgorithmus ALG-1 aus den Vergleichsdatensätzen VDS ein oder mehrere Referenzdatensätze RDS-1, RDS-2, ..., RDS-n ermittelt. Wie z.B. in Zusammenhang mit Figur 2 ausgeführt, weisen die Referenzdatensätze RDS-1, RDS-2, ..., RDS-n eine gewisse Ähnlichkeit mit dem medizinischen Datensatz PDS auf. Dabei wird durch den ersten Datenverarbeitungsalgorithmus ALG-1 ferner für jeden Referenzdatensatz RDS-1, RDS-2, ..., RDS-n jeweils ein Ähnlichkeitsmaß AE-1, AE-2, ..., AE-n ausgegeben, das eine Ähnlichkeit des jeweiligen Referenzdatensatzes RDS-1, RDS-2, ..., RDS-n mit dem medizinischen Datensatz PDS angibt (quantifiziert). Dabei kann der erste Datenverarbeitungsalgorithmus ALG-1 wie in Zusammenhang mit Figur 2 ausgeführt operieren.

In Schritt T30 wird basierend auf den durch die in Schritt T20 ermittelten Referenzdatensätzen RDS-1, RDS-2, ..., RDS-n und den Ähnlichkeitsmaßen AE-1, AE-2, ..., AE-n durch eine fortgesetzte Mensch-Maschine-Interaktion eine medizinische Information MEDI erzeugt. Dabei kann im Wesentlichen wie in Figur 2 beschrieben vorgegangen werden, d.h. die Schritte S30 bis S80 können wiederholt abgearbeitet werden. Ist ein ausreichend gutes Ergebnis, d.h. eine akzeptable medizinische Information MEDI erreicht, kann diese zum weitern Anpassen des ersten Datenauswertungsalgorithmus ALG-1 verwendet werden. Die so erhaltene medizinische Information MEDI ist durch die Nutzereingaben NE optimiert. Medizinische Befunde MD1, MD2, MD3, die sich aufgrund dieser Optimierung als (besonders) relevant für den zu befundenden Patienten herausgestellt haben, legen nahe, dass Referenzdatensätze RDS-1, RDS-2, ..., RDS-n mit solchen medizinischen Befunden ein größere Ähnlichkeit mit dem medizinischer Datensatz PDS aufweisen sollten als andere. Dies kann ausgenutzt werden, um den ersten Datenauswertungsalgorithmus ALG-1 zu überprüfen und ggf. weiter zu trainieren.

Schritt T40 ist entsprechend auf einen Vergleich der Ausgabe des ersten Datenverarbeitungsalgorithmus ALG-1 mit der medizinischen Information MEDI gerichtet. Enthält die medizinische Information MEDI angepasste Ähnlichkeitsmaße, können diese ggf. direkt mit den von dem ersten Datenverarbeitungsalgorithmus ALG-1 bestimmten Ähnlichkeitsmaßen AE-1, AE-2, ..., AE-n verglichen werden. Andernfalls kann z.B. auch aus der relativen Relevanz der medizinischen Befunde MD1, MD2, MD3 in der medizinischen Information MEDI auf mögliche Anpassungen in den Ähnlichkeitsmaßen AE-1, AE-2, ..., AE-n geschlossen werden.

In Schritt T50 erfolgt schließlich eine Anpassung des ersten Datenverarbeitungsalgorithmus ALG-1 basierend auf dem Vergleich aus Schritt T40.

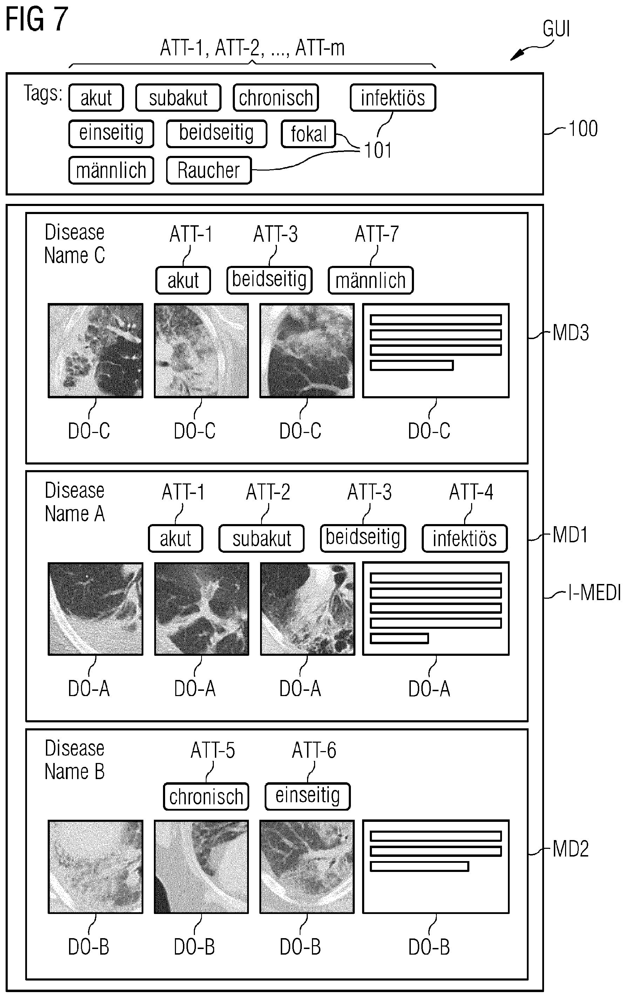

In den **Figuren 7 bis 9** sind beispielhafte Ausführungsformen für grafische Benutzerschnittstellen GUI dargestellt, mit denen eine kontinuierliche Mensch-Maschine-Interaktion nach einigen erfindungsgemäßen Beispielen ausgeführt werden kann.

Nachdem ein Nutzer einen zu befundenden Fall ausgewählt hat, erfolgt zunächst, wie in Zusammenhang mit Figur 2 beschrieben, eine Ähnlichkeitsanalyse. Das Ergebnis der Ähnlichkeitsanalyse sind zum einen die ähnlichen Fälle (in Form eines oder mehrerer Referenzdatensätze RDS-1, RDS-2, ..., RDS-n) und die zugehörigen Ähnlichkeitsmaße AE-1, AE-2, ..., AE-n. Zum anderen werden aus der Ähnlichkeitsanalyse ein oder mehrere medizinische Befunde MD1, MD2, MD3 erhalten, die aufgrund der Ähnlichkeitsanalyse auch für den vorliegenden Fall in Frage kommen. Zudem kann aus der Ähnlichkeitsanalyse eine Information darüber erhalten werden, wie relevant die einzelnen medizinischen Befunde MD1, MD2, MD3 relativ zueinander eingeschätzt werden. So kann aus den Ähnlichkeitsmaße AE-1, AE-2, ..., AE-n beispielsweise eine Rangfolge der medizinischen Befunde MD1, MD2, MD3 für den vorliegenden Fall abgeleitet werden. Diese aus der Ähnlichkeitsanalyse erhaltenen Informationen können zusammenfassend als inzidente medizinischer Information I-MEDI bezeichnet werden. Die inzidente medizinische Information I-MEDI umfasst wenigsten die in der Ähnlichkeitsanalyse ermittelten medizinischen Befunde MD1, MD2, MD3 und vorzugsweise zusätzlich eine Information über deren (relative) Relevanz für den vorliegenden Fall. Darüber hinaus kann die inzidente medizinische Information I-MEDI auch noch weitere Informationen und Daten umfassen, wie z.B. aus den Referenzdatensätze RDS-1, RDS-2, ..., RDS-n oder weiteren Quellen abgeleitete Datenobjekte DO-A, DO-B, DO-C. Diese Datenobjekte können Bilddaten und/oder Textdaten umfassen.

Die inzidente medizinische Information I-MEDI kann dem Nutzer mit einer beispielhaft in Figur 7 dargestellten grafischen Nutzerschnittstelle GUI angezeigt werden. Die dabei verwendete Darstellungsform ist dabei bevorzugt derart ausgebildet, dass eine aus der Ähnlichkeitsanalyse ermittelte Rangfolge der medizinischen Befunde MD1, MD2, MD3 für den Nutzer ersichtlich ist. In den in den Figuren 7 bis 9 gezeigten Beispielen wird dies durch die Darstellung der medizinischen Befunde MD1, MD2, MD3 in einer Liste bewerkstelligt, die nach abnehmender relativer Relevanz gestaffelt ist. Der für den zu befundenden Fall auf Grundlage der Ähnlichkeitsanalyse zunächst relevanteste medizinische Befund wäre im in Figur 7 gezeigten Beispiel folglich der medizinische Befund MD3. Alternativ oder zusätzlich kann die relative Relevanz für jeden medizinischen Befund MD1, MD2, MD3 durch die Visualisierung eines Konfidenzwertes dargestellt werden, welcher Konfidenzwert beispielsweise aus den Ähnlichkeitsmaße AE-1, AE-2, ..., AE-n abgeleitet werden kann. Dies hat den weiteren Effekt, dass der Nutzer von dem System 1 eine Angabe darüber erhält, wie eindeutig die Rangfolge ist. Als weitere Option kann die Rangfolge zusätzlich oder alternativ durch die Anzeige der jeweiligen Rangnummer kenntlich gemacht werden.

Die medizinischen Befunde MD1, MD2, MD3 können sich insbesondere auf Krankheiten beziehen, die für die Referenzdatensätze RDS-1, RDS-2, ..., RDS-n diagnostiziert wurden. Entsprechend kann bei der Darstellung der I-MEDI in der grafischen Benutzerschnittstelle GUI der Name der jeweiligen Krankheit angegeben werden. Dabei können alle mit der Ähnlichkeitsanalyse gefundenen medizinischen Befunde MD1, MD2, MD3 dargestellt werden oder nur ein Teil - etwa die Relevantesten. Weitere medizinische Befunde kann sich der Nutzer dann z.B. durch "Blättern" in der Listendarstellung anzeigen lassen.

Zusätzlich zu den Namen der Krankheiten (oder beliebigen anderen Identifikatoren der medizinischen Befunde) können in der inzidenten medizinischen Information I-MEDI weitere Datenobjekte DO-A, DO-B, DO-C enthalten sein und entsprechend angezeigt werden. Die Datenobjekte DO-A, DO-B, DO-C können beispielsweise aus den Referenzdatensätzen RDS-1, RDS-2, ..., RDS-n erzeugt werden. Insbesondere können solche aus den Referenzdatensätzen RDS-1, RDS-2, ..., RDS-n erzeugte Datenobjekte DO-A, DO-B, DO-C Bilddaten umfassen, die dann als Bilder in der grafischen Benutzerschnittstelle GUI dargestellt werden (vgl. Figuren 7 bis 9). Ferner können die Datenobjekte DO-A, DO-B, DO-C aus anderen Quellen, wie z.B. medizinischen Richtlinien oder elektronischen Kompendien oder Lehrbüchern extrahiert werden, wie z.B. eRef von Thieme oder Radiopedia. Insbesondere können sich solche aus anderen Quellen erzeugte Datenobjekte DO-A, DO-B, DO-C auf die medizinischen Befunde MD1, MD2, MD3 beziehen und z.B. nähere Informationen zu der zugrundeliegenden Krankheit bereitstellen. Diese Datenobjekte können Bilddaten und/oder, wie in den Figuren 7 bis 9 schematisch dargestellt, Textdaten enthalten. Die Datenobjekte DO-A, DO-B, DO-C werden für die Anzeige der I-MEDI bevorzugt so dargestellt, dass der Nutzer sie visuell zu dem jeweils zugehörigen medizinischen Befund MD1, MD2, MD3 zuordnen kann. Wie in den Figuren 7 bis 9 gezeigt, können die Datenobjekte DO-A, DO-B, DO-C dazu entsprechend gruppiert werden.

Wie im Detail in Zusammenhang mit Figur 2 erläutert, sieht das erfindungsgemäße Verfahren vor, den medizinischen Befunden MD1, MD2, MD3 jeweils ein oder mehrere medizinischen Attribute ATT-1, ATT-2, ..., ATT-m zuzuordnen. Die jeweiligen medizinischen Attribute ATT-1, ATT-2, ..., ATT-m und ihre Zuordnung zum jeweiligen medizinischen Befund kann dabei in der I-MEDI enthalten sein. Die einem medizinischen Befund MD1, MD2, MD3 zugeordneten medizinischen Attribute ATT-1, ATT-2, ..., ATT-m können zusammen mit dem jeweiligen medizinischen Befund dargestellt werden, und zwar insbesondere derart, dass die Zuordnung für den Nutzer ersichtlich wird (vgl. Figuren 7 bis 9).

Wie in den Figuren 7 bis 9 gezeigt, können die medizinischen Attribute ATT-1, ATT-2, ..., ATT-m als Worte dargestellt sein, welche Eigenschaften bzw. Kennzeichen des jeweiligen medizinischen Befund MD1, MD2, MD3 und/oder der Referenzdatensätze RDS-1, RDS-2, ..., RDS-n beschreiben. Alternativ oder zusätzlich können auch Zeichen, Symbole oder Wortgruppen zur Darstellung der medizinischen Attribute ATT-1, ATT-2, ..., ATT-m verwendet werden.

Zusätzlich werden die medizinischen Attribute ATT-1, ATT-2, ..., ATT-m in einer Schaltfläche 100 dargestellt. In der Schaltfläche 100 können die medizinischen Attribute ATT-1, ATT-2, ..., ATT-m jeweils mit einem Schaltelement 101 verknüpft sein, mit dem der Nutzer eine auf das jeweilige medizinischen Attribute ATT-1, ATT-2, ..., ATT-m bezogene Nutzereingabe NE vornehmen kann. Zur einfacheren Bezugnahme für den Nutzer kann die Schaltfläche 100 mit dem Wort "Tags" o.Ä. bezeichnet sein. In der Schaltfläche 100 sind insbesondere mit den medizinischen Befunden MD1, MD2, MD3 verknüpfte medizinischen Attribute ATT-1, ATT-2, ..., ATT-m dargestellt. Dabei können alle mit den medizinischen Befunden MD1, MD2, MD3 verknüpfte medizinischen Attribute ATT-1, ATT-2, ..., ATT-m oder nur eine Auswahl der verknüpften medizinischen Attribute ATT-1, ATT-2, ..., ATT-m dargestellt sein. Weiterhin können alle überhaupt vorgesehenen bzw. möglichen medizinischen Attribute ATT-1, ATT-2, ..., ATT-m, beispielsweise alle in der Zuordnungsvorschrift enthaltenen medizinischen Attribute ATT-1, ATT-2, ..., ATT-m, dargestellt sein. Mit anderen Worten können in der Schaltfläche 100 der grafischen Benutzerschnittstelle GUI unabhängig von dem Fall (also insbesondere unabhängig von den durch die Ähnlichkeitsanalyse bestimmten medizinischen befunden MD1, MD2, MD3) auch stets die gleichen medizinischen Attribute ATT-1, ATT-2, ..., ATT-m dargestellt werden. Die medizinischen Attribute ATT-1, ATT-2, ..., ATT-m können der besseren Übersichtlichkeit halber nach Kategorien gruppiert dargestellt sein. In dem gezeigten Beispiel sind beispielsweise die den Krankheitsverlauf charakterisierenden medizinischen Attribute "akut", "subakut" und "chronisch" ebenso gruppiert wie die den Auftrittsort der Erkrankung kennzeichnenden medizinischen Attribute "einseitig", "beidseitig" und "fokal". Daneben können die medizinischen Attribute ATT-1, ATT-2, ..., ATT-m nach Relevanz bzw. ihrer potentiell diskriminierenden Wirkung angeordnet werden.

Die Schaltfläche 100 bzw. die Schaltelemente 101 sind derart ausgebildet, dass der Nutzer eine einzelne medizinischen Attribute ATT-1, ATT-2, ..., ATT-m betreffende Nutzereingabe NE mittels der grafischen Benutzerschnittstelle GUI eingeben kann. Eine Nutzereingabe NE kann dabei insbesondere eine Verifizierung einzelner medizinischen Attribute ATT-1, ATT-2, ..., ATT-m umfassen. Eine Verifizierung kann beispielsweise eine Auswahl bzw. Bestätigung eines oder mehrerer medizinischer Attribute für den zu befundenden Fall umfassen. Ferner kann eine Verifizierung eine Abwahl bzw. ein Ausschließen eines oder mehrerer medizinischen Attribute ATT-1, ATT-2, ..., ATT-m für den zu befundenden Fall umfassen. Dazu kann das Schaltelement 101 beispielsweise als Taster implementiert sein, bei dem ein einfaches Betätigen (beispielsweise durch Mausklick) eine Auswahl und zweifaches Betätigen (beispielsweise durch Doppelklick) eine Abwahl des jeweiligen medizinischen Attributs bedeuten. Alternativ können eine Eingabemöglich durch ein oder mehrere "Radiobuttons" und/oder andere Lösungen implementiert sein. Ferner kann auch vorgesehen sein, dem Nutzer eine differenziertere Möglichkeit der Nutzereingabe NE bereitzustellen, mit dem sie oder er beispielsweise einstellen kann, in welchem Maß einzelne medizinischen Attribute ATT-1, ATT-2, ..., ATT-m zutreffen. Im Rahmen der grafischen Benutzerschnittstelle GUI kann dies z.B. durch die Bereitstellung von Schiebereglern implementiert sein.

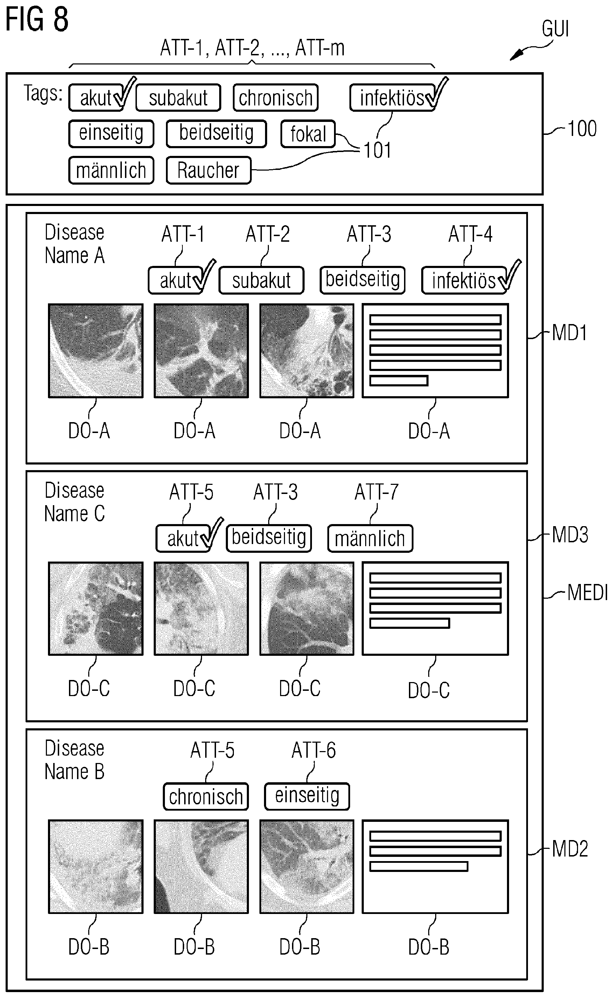

In Figur 8 ist beispielhaft dargestellt, wie die grafische Benutzerschnittstelle GUI in Reaktion auf eine Nutzereingabe NE angepasst werden kann. Im vorliegenden Beispiel hat der Nutzer die medizinischen Attribute "akut" und "infektiös" über die Schaltfläche 100 bestätigt. Zur besseren Orientierung kann diese Eingabe grafisch kenntlich gemacht werden (wie in Figur 8 beispielhaft durch Haken illustriert). Entsprechend können auch die medizinischen Attribute ATT-1, ATT-2, ..., ATT-m bei den jeweiligen medizinischen Befunden MD1, MD2, MD3 gekennzeichnet werden. Durch die Nutzereingabe NE werden jene medizinischen Befunde MD1, MD2, MD3 relevanter, die ein oder mehrere der ausgewählten medizinischen Attribute ATT-1, ATT-2, ..., ATT-m zeigen. Infolge der Nutzereingabe NE wird deshalb die medizinische Information MEDI neu bestimmt bzw. angepasst (vgl. Ausführungen zu Figur 2) und die Darstellung in der grafischen Benutzerschnittstelle GUI wird nachgeführt. Im gezeigten Beispiel hat dies zur Folge, dass die medizinischen Befunde MD1 und MD3 den Listenplatz tauschen, weil der medizinischen Befund MD1 infolge der Nutzereingabe NE nun als relevanter als der medizinischen Befund MD3 einzuschätzen ist. Etwaige gezeigte Datenobjekte DO-A, DO-B, DO-C werden falls erforderlich nachgeführt.

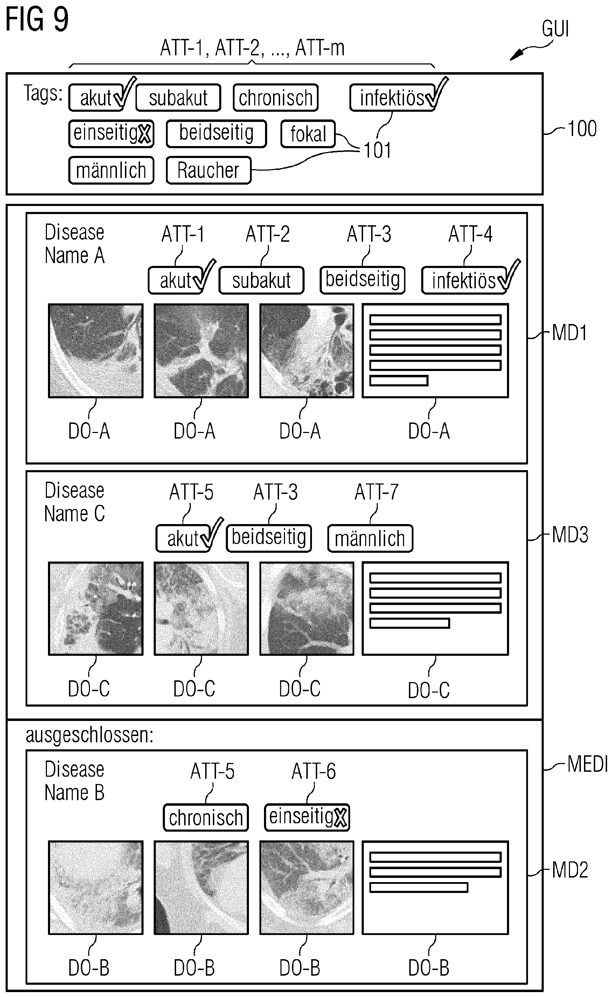

In Figur 9 ist beispielhaft dargestellt, wie die grafische Benutzeroberfläche GUI in Reaktion auf eine Nutzereingabe NE angepasst werden kann, falls die Nutzereingabe NE zusätzlich noch den Ausschluss eines medizinischen Attributs ATT-1, ATT-2, ..., ATT-m enthält. Im gezeigten Beispiel kann der Nutzer das medizinische Attribut "einseitig" für den ihr oder ihm vorliegenden Fall ausschließen. Damit wird der medizinische Befund MD2 für den zu befundenden Fall irrelevant, da er dieses medizinische Attribut voraussetzt. Die medizinische Information MEDI wird entsprechend angepasst. In der grafischen Benutzerschnittstelle GUI kann dies beispielsweise dadurch umgesetzt werden, dass der medizinische Befund MD2 als "ausgeschlossen" gekennzeichnet wird.

Während Ausführungsbeispiele insbesondere unter Bezugnahme auf die Figuren detailliert beschrieben wurden, sei darauf hingewiesen, dass eine Vielzahl von Abwandlungen möglich ist. Außerdem sei darauf hingewiesen, dass es sich bei den exemplarischen Ausführungen lediglich um Beispiele handelt, die den Schutzbereich, die Anwendung und den Aufbau in keiner Weise einschränken sollen. Vielmehr wird dem Fachmann durch die vorausgehende Beschreibung ein Leitfaden für die Umsetzung von mindestens einem Ausführungsbeispiel gegeben, wobei diverse Abwandlungen, insbesondere alternative oder zusätzliche Merkmale und/oder Abwandlung der Funktion und/oder Anordnung der beschriebenen Bestandteile, nach Wunsch des Fachmanns vorgenommen werden können, ohne dass dabei von dem in den angehängten Ansprüchen jeweils festgelegten Gegenstand sowie seiner rechtlichen Äquivalent abgewichen wird und/oder deren Schutzbereich verlassen wird.

## Patentansprüche

1. Computerimplementiertes Verfahren zur Bereitstellung einer medizinischen Information (MEDI) mit den Schritten:
Empfangen (S10) eines medizinischen Datensatzes eines Patienten;
Ermitteln (S20) eines oder mehrerer Referenzdatensätze (RDS-1, RDs-2, ..., RDS-n) aus mehreren Vergleichsdatensätzen (VDS) basierend auf Ähnlichkeitsmaßen (AE-1, AE-2, ..., AE-n), wobei ein Ähnlichkeitsmaß (AE-1, AE-2, ..., AE-n) auf einer Ähnlichkeit zwischen dem medizinischen Datensatz (PDS) und einem Vergleichsdatensatz (VDS) basiert, und wobei jeder Vergleichsdatensatz (VDS) mit wenigstens einem vorbekannten medizinischen Befund (MD1, MD2, MD3) assoziiert ist;
Identifizieren (S40) eines oder mehrerer medizinischer Attribute (ATT-1, ATT-2, ..., ATT-m), welche medizinischen Attribute (ATT-1, ATT-2, ..., ATT-m) jeweils ein Kennzeichen eines oder mehrerer der mit den Referenzdatensätzen (RDS-1, RDs-2,..., RDS-n) assoziierten vorbekannten medizinischen Befunde (MD1, MD2, MD3) umfassen, wobei der Schritt des Identifizierens (S40) der ein oder mehreren medizinischen Attribute ein Bestimmen einer Attribut-Rangfolge basierend auf den für die jeweiligen Referenzdatensätze (RDS-1, RDS-2, ..., RDS-n) bestimmten Ähnlichkeitsmaßen (AE-1, AE-2, ..., AE-n) und/oder basierend auf einer diskriminierenden Wirkung der medizinischen Attribute (ATT-1, ATT-2, ..., ATT-m) hinsichtlich der Relevanz der medizinischen Befunde (MD1, MD2, MD3) für den zu befundenden Patienten und/oder basierend auf einer oder mehreren verschiedenen Attribut-Kategorien umfasst;
Anzeigen (S50) der identifizierten medizinischen Attribute (ATT-1, ATT-2, ..., ATT-m) über eine Nutzerschnittstelle (10), wobei das Anzeigen (S50) der identifizierten medizinischen Attribute (ATT-1, ATT-2, ..., ATT-m) in einer Darstellungsform (GUI) erfolgt, die derart ausgestaltet ist, dass der Nutzer die Attribut-Rangfolge wahrnehmen kann;
Empfangen (S60) einer Nutzereingabe (NE) eines Nutzers betreffend die angezeigten medizinischen Attribute (ATT-1, ATT-2, ..., ATT-m) über die Nutzerschnittstelle (10), wobei die Nutzereingabe (NE) eine Verifikation wenigstens eines Teils der identifizierten medizinischen Attribute (ATT-1, ATT-2, ..., ATT-m) umfassend ein Bestätigen und/oder Verwerfen eines oder mehrerer der identifizierten Attribute (ATT-1, ATT-2, ..., ATT-m) aufweist;
Erzeugen (S70) einer medizinischen Information (MEDI) für den Patienten basierend auf den mit den Referenzdatensätzen (RDS-1, RDs-2, ..., RDS-n) assoziierten medizinischen Befunden (MD1, MD2, MD3) sowie der Nutzereingabe (NE) umfassend ein Bestimmen einer Rangfolge wenigstens eines Teils der mit den Referenzdatensätzen (RDS-1, RDs-2, ..., RDS-n) assoziierten vorbekannten medizinischen Befunde (MD1, MD2, MD3) basierend auf den für die jeweiligen Referenzdatensätze (RDS-1, RDs-2, ..., RDS-n) bestimmten Ähnlichkeiten (AE-1, AE-2, ..., AE-n) und basierend auf der Nutzereingabe (NE); und
Bereitstellen (S80) der medizinischen Information (MEDI).

2. Verfahren nach Anspruch 1, bei dem
das Bereitstellen (S80) der medizinischen Information (MEDI) ein Anzeigen (S81) der medizinischen Information (MEDI) über die Nutzerschnittstelle (10) umfasst; wobei
das Anzeigen (S81) der medizinischen Information (MEDI) insbesondere in einer Darstellungsform (GUI) erfolgt, die derart ausgebildet ist, dass der Nutzer die Rangfolge der medizinischen Befunde (MD1, MD2, MD3) wahrnehmen kann.

3. Verfahren nach einem der vorhergehenden Ansprüche, bei dem
die medizinischen Attribute (ATT-1, ATT-2, ..., ATT-m) eine oder mehrere der folgenden Angaben umfassen:
eine, insbesondere demografische, Angabe bezüglich einer mit dem klinischen Befund (MD1, MD2, MD3) assoziierten Patientengruppe;
eine Angabe bezüglich eines oder mehrerer mit dem jeweiligen medizinischen Befund (MD1, MD2, MD3) assoziierter Symptome;
eine Angabe bezüglich eines oder mehrerer mit dem jeweiligen medizinischen Befund (MD1, MD2, MD3) assoziierter Differentialdiagnosen;
eine Angabe bezüglich eines oder mehrerer mit dem jeweiligen medizinischen Befund (MD1, MD2, MD3) assoziierter klinischer Anzeichen; und/oder
eine Angabe bezüglich eines oder mehrerer mit dem jeweiligen medizinischen Befund (MD1, MD2, MD3) assoziierter klinischer Gegenanzeichen.

4. Verfahren nach einem der vorhergehenden Ansprüche, bei dem
der Schritt des Identifizierens (S40) der ein oder mehreren medizinischen Attribute (ATT-1, ATT-2, ..., ATT-m) umfasst:
Bereitstellen einer Zuordnung (S41), die wenigstens einem vorbekannten medizinischen Befund (MD1, MD2, MD3) ein oder mehrere medizinische Attribute (ATT-1, ATT-2, ..., ATT-m) zuordnet; und
Zuordnen (S42) eines oder mehrerer verschiedener medizinischer Attribute (ATT-1, ATT-2, ..., ATT-m) zu wenigstem einem der mit den Referenzdatensätzen (RDS-1, RDS-2, ..., RDS-n) assoziierten medizinischen Befunde (MD1, MD2, MD3) unter Verwendung der Zuordnung.

5. Verfahren nach einem der vorhergehenden Ansprüche ferner mit dem Schritt:
Erzeugen (S30) einer inzidenten medizinischen Information (I-MEDI) basierend auf den mit den Referenzdatensätzen (RDS-1, RDS-2, ..., RDS-n) assoziierten medizinischen Befunden (MD1, MD2, MD3), insbesondere basierend auf den für die jeweiligen Referenzdatensätze (RDS-1, RDS-2, ..., RDS-n) bestimmten Ähnlichkeiten (AE-1, AE-2, ..., AE-n), wobei
der Schritt des Erzeugens (S70) der medizinischen Information (MEDI) ein Anpassen der inzidenten medizinischen Information (I-MEDI) basierend auf der Nutzereingabe (NE) umfasst.

6. Verfahren nach einem der vorhergehenden Ansprüche, bei dem
der medizinische Datensatz (PDS) und die Vergleichsdatensätze (VDS) jeweils medizinische Bilddaten umfassen; und
ein Ähnlichkeitsmaß (AE-1, AE-2, ..., AE-n) jeweils auf einer Ähnlichkeit zwischen den medizinischen Bilddaten des medizinischen Datensatzes (PDS) und den medizinischen Bilddaten eines Vergleichsdatensatzes (VDS) basiert.

7. Verfahren nach einem der vorhergehenden Ansprüche, bei dem
das Ermitteln (S20) der ein oder mehreren Referenzdatensätze (RDS-1, RDS-2, ..., RDS-n) umfasst:
Extrahieren (S21) eines Datendeskriptors aus dem medizinischen Datensatz (PDS);
Empfangen (S22) jeweils eines korrespondierenden Datendeskriptors zu jedem der Vergleichsdatensätze (VDS);
Bestimmen (S23), für jeden Vergleichsdatensatz (VDS), eines Ähnlichkeitsmaßes, wobei ein Ähnlichkeitsmaß jeweils auf einer Ähnlichkeit zwischen dem Datendeskriptor und einem korrespondierenden Datendeskriptor basiert; und
Ermitteln (S24) der ein oder mehrerer Referenzdatensätze (RDS-1, RDS-2, ..., RDS-n) basierend auf den bestimmten Ähnlichkeitsmaßen.

8. Verfahren nach einem der vorhergehenden Ansprüche, bei dem
das Ermitteln (S20) der ein oder mehreren Referenzdatensätze (RDS-1, RDS-2, ..., RDS-n) ein Anwenden einer trainierten Funktion (ALG-1) umfasst, welche trainierte Funktion (ALG-1) dazu ausgebildet ist, ein Ähnlichkeitsmaß (AE-1, AE-2, ..., AEn) zwischen medizinischen Datensätzen (PDS, VDS) zu bestimmen.

9. Verfahren nach Anspruch 8, bei dem
der Schritt des Bereitstellens (S80) umfasst:
Bereitstellen (S81) der medizinischen Information (MEDI) an die trainierte Funktion (ALG-1);
ferner mit dem Schritt:
Anpassen (T40, T50) der trainierten Funktion (ALG-1) basierend auf der medizinischen Information (MEDI).

10. System (1) zur Bereitstellung einer medizinischen Information (MEDI) umfassend:
eine Schnittstelle (10, 30) und eine Steuerung (40)
wobei die Steuerung (40) dazu ausgebildete ist:
- einen medizinischen Datensatzes (PDS) eines Patienten über die Schnittstelle (10, 30) zu empfangen;
- einen oder mehrere Referenzdatensätze (RDS-1, RDs-2, ..., RDS-n) aus mehreren Vergleichsdatensätze (VDS) basierend auf Ähnlichkeitsmaßen (AE-1, AE-2, ..., AE-n) zu ermitteln, wobei ein Ähnlichkeitsmaß (AE-1, AE-2, ..., AE-n) auf einer Ähnlichkeit zwischen dem medizinischen Datensatz (PDS) und einem Vergleichsdatensatz (VDS) basiert, und wobei jeder Vergleichsdatensatz (VDS) mit wenigstens einem vorbekannten medizinischen Befund (MD1, MD2, MD3) assoziiert ist;
- ein oder mehrere medizinische Attribute (ATT-1, ATT-2, ..., ATT-m) zu identifizieren, welche medizinischen Attribute (ATT-1, ATT-2, ..., ATT-m) jeweils ein Kennzeichen eines oder mehrerer der mit den Referenzdatensätzen (RDS-1, RDs-2,..., RDS-n) assoziierten vorbekannten medizinischen Befunde (MD1, MD2, MD3) umfassen, und eine Attribut-Rangfolge basierend auf den für die jeweiligen Referenzdatensätze (RDS-1, RDS-2, ..., RDS-n) bestimmten Ähnlichkeitsmaßen (AE-1, AE-2, ..., AE-n) und/oder basierend auf einer diskriminierenden Wirkung der medizinischen Attribute (ATT-1, ATT-2, ..., ATT-m) hinsichtlich der Relevanz der medizinischen Befunde (MD1, MD2, MD3) für den zu befundenden Patienten und/oder basierend auf einer oder mehreren verschiedenen Attribut-Kategorien zu bestimmen;
- die identifizierten medizinischen Attribute (ATT-1, ATT-2, ..., ATT-m) über die Schnittstelle (10, 30) in einer Darstellungsform anzuzeigen, die derart ausgestaltet ist, dass der Nutzer die Attribut-Rangfolge wahrnehmen kann;
- eine Nutzereingabe (NE) eines Nutzers betreffend die angezeigten medizinischen Attribute (ATT-1, ATT-2, ..., ATT-m) über die Schnittstelle (10, 30) zu empfangen, wobei die Nutzereingabe (NE) eine Verifikation wenigstens eines Teils der identifizierten medizinischen Attribute (ATT-1, ATT-2, ..., ATT-m) umfassend ein Bestätigen und/oder Verwerfen eines oder mehrerer der identifizierten Attribute (ATT-1, ATT-2, ..., ATT-m) aufweist;
- eine medizinische Information (MEDI) für den Patienten basierend auf den mit den Referenzdatensätzen (RDS-1, RDS-2, ..., RDS-n) assoziierten medizinischen Befunden (MD1, MD2, MD3) sowie der Nutzereingabe (NE) zu erzeugen umfassend ein Bestimmen einer Rangfolge wenigstens eines Teils der mit den Referenzdatensätzen (RDS-1, RDs-2, ..., RDS-n) assoziierten vorbekannten medizinischen Befunde (MD1, MD2, MD3) basierend auf den für die jeweiligen Referenzdatensätze (RDS-1, RDs-2, ..., RDS-n) bestimmten Ähnlichkeiten (AE-1, AE-2, ..., AE-n) und basierend auf der Nutzereingabe (NE); und
- die medizinische Information (MEDI) über die Schnittstelle (10, 30) bereitzustellen.

11. Computerprogrammprodukt, welches ein Programm umfasst und direkt in einen Speicher einer programmierbaren Recheneinheit einer Steuerung (40) ladbar ist, mit Programmmitteln, um ein Verfahren nach den Ansprüchen 1 bis 9 auszuführen, wenn das Programm in der Steuerung (40) ausgeführt wird.

12. Computerlesbares Speichermedium, auf welchem lesbare und ausführbare Programmabschnitte gespeichert sind, um alle Schritte der Verfahren nach einem der Ansprüche 1 bis 9 auszuführen, wenn die Programmabschnitte von der Steuerung (40) ausgeführt werden.

## Claims

1. Computer-implemented method for the provision of an item of medical information (MEDI) with the steps:
receiving (S10) a medical data record of a patient;
determining (S20) one or more reference data records (RDS-1, RDs-2, ..., RDS-n) from a number of comparison data records (VDS) based on degrees of similarity (AE-1, AE-2, ..., AE-n),
wherein a degree of similarity (AE-1, AE-2, ..., AE-n) is based on a similarity between the medical data record (PDS) and a comparison data record (VDS), and wherein each comparison data record (VDS) is associated with at least one already known medical finding (MD1, MD2, MD3);
identifying (S40) one or more medical attributes (ATT-1, ATT-2, ..., ATT-m), which medical attributes (ATT-1, ATT-2, ..., ATT-m) comprise in each case an identifier of one or more of the already known medical findings (MD1, MD2, MD3) associated with the reference data records (RDS-1, RDs-2), ...,RDS-n), wherein the step of identifying (S40) the one or more medical attributes comprises a determination of an attribute ranking based on the degrees of similarity (AE-1, AE-2, ..., AE-n) determined for the respective reference data records (RDS-1, RDS-2, ..., RDS-n) and/or based on a discriminatory effect of the medical attributes (ATT-1, ATT-2, ..., ATT-m) with respect to the relevance of the medical findings (MD1, MD2, MD3) for the patient to be diagnosed and/or based on one or more different attribute categories;
displaying (S50) the identified medical attributes (ATT-1, ATT-2, ..., ATT-m) by way of a user interface (10), wherein the display (S50) of the identified medical attributes (ATT-1, ATT-2, ..., ATT-m) takes place in a display format (GUI) which is configured so that the user can perceive the attribute ranking;
receiving (S60) a user input (NE) of a user relating to the displayed medical attributes (ATT-1, ATT-2, **...,** ATT-m) by way of the user interface (10), wherein the user input (NE) has a verification of at least one part of the identified medical attributes (ATT-1, ATT-2, **...,** ATT-m) comprising a confirmation and/or rejection of one or more of the identified attributes (ATT-1, ATT-2, **...,** ATT-m);
generating (S70) an item of medical information (MEDI) for the patient based on the medical findings (MD1, MD2, MD3) associated with the reference data records (RDS-1, RDs-2, ..., RDS-n) and the user input (NE) comprising a determination of a ranking of at least one part of the already known medical findings (MD1, MD2, MD3) associated with the reference data records (RDS-1, RDs-2, ..., RDS-n) based on the similarities (AE-1, AE-2, ..., AE-n) determined for the respective reference data records (RDS-1, RDs-2, ..., RDS-n) and based on the user input (NE); and
providing (S80) the medical information (MEDI).

2. Method according to claim 1, in which
the provision (S80) of the medical information (MEDI) comprises displaying (S81) the medical information (MEDI) by way of the user interface (10); wherein
the display (S81) of the medical information (MEDI) takes in place in particular in a display format (GUI) which is embodied such that the user can perceive the ranking of the medical findings (MD1, MD2, MD3).

3. Method according to one of the preceding claims, in which the medical attributes (ATT-1, ATT-2, ..., ATT-m) comprise one or more of the following statements:
an, in particular demographic, statement relating to a patient group associated with the clinical findings (MD1, MD2, MD3);
a statement relating to one or more symptoms associated with the respective medical findings (MD1, MD2, MD3);
a statement relating to one or more differential diagnoses associated with the respective medical findings (MD1, MD2, MD3);
a statement relating to one or more clinical markers associated with the respective medical findings (MD1, MD2, MD3); and/or
a statement relating to one or more clinical contra-markers associated with the respective medical findings (MD1, MD2, MD3).

4. Method according to one of the preceding claims, in which
the step of identifying (S40) the one or more medical attributes (ATT-1, ATT-2, ..., ATT-m) comprises:
providing an assignment (S41), which assigns one or more medical attributes (ATT-1, ATT-2, ..., ATT-m) to at least one already known medical finding (MD1, MD2, MD3); and
assigning (S42) one or more different medical attributes (ATT-1, ATT-2, ..., ATT-m) to at least one of the medical findings (MD1, MD2, MD3) associated with the reference data records (RDS-1, RDS-2, ..., RDS-n) using the assignment.

5. Method according to one of the preceding claims further with the step:
generating (S30) an item of incident medical information (I-MEDI) based on the medical findings (MD1, MD2, MD3) associated with the reference data records (RDS-1, RDS-2, ..., RDS-n), in particular based on the similarities (AE-1, AE-2, ..., AE-n) determined for the respective reference data records (RDS-1, RDS-2, ..., RDS-n), wherein
the step of generating (S70) the medical information (MEDI) comprises an adjustment of the incident medical information (I-MEDI) based on the user input (NE).

6. Method according to one of the preceding claims, in which
the medical data record (PDS) and the comparison data records (VDS) each comprise medical image data; and
a degree of similarity (AE-1, AE-2, ..., AE-n) is based in each case on a similarity between the medical image data of the medical data record (PDS) and the medical image data of a comparison data record (VDS).

7. Method according to one of the preceding claims, in which
the determination (S20) of the one or more reference data records (RDS-1, RDS-2, ..., RDS-n) comprises:
extracting (S21) a data descriptor from the medical data record (PDS);
receiving (S22) in each case a corresponding data descriptor for each of the comparison data records (VDS);
determining (S23), for each comparison data record (VDS), a degree of similarity, wherein a degree of similarity is based in each case on a similarity between the data descriptor and a corresponding data descriptor; and
determining (S24) the one or more reference data records (RDS-1, RDS-2, ..., RDS-n) based on the determined degrees of similarity.

8. Method according to one of the preceding claims, in which
the determination (S20) of the one or more reference data records (RDS-1, RDS-2, ..., RDS-n) comprises an application of a trained function (ALG-1), which trained function (ALG-1) is embodied to determine a degree of similarity (AE-1, AE-2, ... AE-n) between medical data records (PDS, VDS).

9. Method according to claim 8, in which
the step of providing (S80) comprises:
providing (S81) the medical information (MEDI) to the trained function (ALG-1);
further with the step:
adjusting (T40, T50) the trained function (ALG-1) based on the medical information (MEDI).

10. System (1) for providing an item of medical information (MEDI) comprising:
an interface (10, 30) and a controller (40)
wherein the controller (40) is embodied:
- to receive a medical data record (PDS) of a patient by way of the interface (10, 30);
- to determine one or more reference data records (RDS-1, RDs-2, ..., RDS-n) from a number of comparison data records (VDS) based on degrees of similarity (AE-1, AE-2, **..,** AE-n), wherein a degree of similarity (AE-1, AE-2, ..., AE-n) is based on a similarity between the medical data record (PDS) and a comparison data record (VDS), and wherein each comparison data record (VDS) is associated with at least one already known medical finding (MD1, MD2, MD3);
- to identify one or more medical attributes (ATT-1, ATT-2, ..., ATT-m), which medical attributes (ATT-1, ATT-2, ..., ATT-m) in each case comprise an identifier of one or more of the already known medical findings (MD1, MD2, MD3) associated with the reference data records (RDS-1, RDs-2, ..., RDS-n) and to determine an attribute ranking based on the degrees of similarity (AE-1, AE-2, ..., AE-n) determined for the respective reference data records (RDS-1, RDS-2, ..., RDS-n) and/or based on a discriminating effect of the medical attributes (ATT-1, ATT-2, ..., ATT-m) in respect of the relevance of the medical findings (MD1, MD2, MD3) for the patient to be diagnosed and/or based on one or more different attribute categories;
- to display the identified medical attributes (ATT-1, ATT-2, ..., ATT-m) by way of the interface (10, 30) in a display format which is configured so that the user can perceive the attribute ranking;
- to receive a user input (NE) of a user relating to the indicated medical attributes (ATT-1, ATT-2, ..., ATT-m,) by way of the interface (10, 30), wherein the user input (NE) has a verification of at least one part of the identified medical attributes (ATT-1, ATT-2, ..., ATT-m) comprising a confirmation and/or rejection of one or more of the identified attributes (ATT-1, ATT-2, ..., ATT-m);
- to generate an item of medical information (MEDI) for the patient based on the medical findings (MD1, MD2, MD3) associated with the reference data records (RDS-1, RDS-2, ..., RDS-n) and the user input (NE) comprising a determination of a ranking of at least one part of the already known medical findings (MD1, MD2, MD3) associated with the reference data records (RDS-1, RDs-2, ..., RDS-n) based on the similarities (AE-1, AE-2, ..., AE-n) determined for the respective reference data records (RDS-1, RDs-2, ..., RDS-n) and based on the user input (NE); and
- to provide the medical information (MEDI) by way of the interface (10, 30).

11. Computer program product which comprises a program and can be loaded directly into a memory of a programmable computing unit of a controller (40), having program means, in order to execute a method according to claims 1 to 9, when the program is executed in the controller (40).

12. Computer-readable storage medium, on which readable and executable program sections are stored, in order to execute all steps of the method according to one of claims 1 to 9, when the program sections are executed by the controller (40).

## Revendications

1. Procédé mis en œuvre par ordinateur pour disposer d'une information (MEDI) médicale comprenant les stades :
réception (S10) d'un ensemble de données médicales d'un patient ;
détermination (S20) d'un ou de plusieurs ensembles (RDS-1, RDs-2, ..., RDS-n) de données de référence à partir de plusieurs ensembles (VDS) de données de comparaison reposant sur des mesures (AE-1, AE-2, ..., AE-n) de similarité, dans lequel une mesure (AE-1, AE-2, ..., AE-n) de similarité repose sur une similarité entre l'ensemble (PDS) de données médicales et un ensemble (VDS) de données de comparaison, et dans lequel chaque ensemble (VDS) de données de comparaison est associé à au moins une constatation (MD1, MD2, MD3) médicale déjà connue ;
identification (S40) d'un ou de plusieurs attributs (ATT-1, ATT-2,..., ATT-m) médicaux, lesquels attributs (ATT-1, ATT-2, ..., ATT-m) médicaux comprennent respectivement une caractéristique d'une ou de plusieurs des constatations (MD1, MD2, MD3) médicales déjà connues associées aux ensembles (RDS-1, RDs-2,..., RDS-n) de données de référence, dans lequel le stade de l'identification (S40) du un ou des plusieurs attributs médicaux comprend une détermination d'une succession de rang d'attribut reposant sur les mesures (AE-1, AE-2, ..., AE-n) de similarité déterminées pour les ensembles (RDS-1, RDS-2, ..., RDS-n) de données de référence respectifs et/ou reposant sur un effet discriminant des attributs (ATT-1, ATT-2, ..., ATT-m) médicaux en ce qui concerne la pertinence des constatations (MD1, MD2, MD3) médicales pour le patient à étudier et/ou sur la base d'une ou de plusieurs catégories d'attribut différentes ;
affichage (S50) des attributs (ATT-1, ATT-2, ..., ATT-m) médicaux identifiés par une interface (10) d'utilisateur, dans lequel l'affichage (S50) des attributs (ATT-1, ATT-2, ..., ATT-m) médicaux identifiés s'effectue sous une forme (GUI) de représentation, qui est conformée de manière à ce que l'utilisateur puisse percevoir la succession de rang d'attribut ;
réception (S60) d'une entrée (NE) d'un utilisateur concernant les attributs (ATT-1, ATT-2, ..., ATT-m) médicaux affichés en passant par l'interface (10) d'utilisateur, dans lequel l'entrée (NE) d'utilisateur comporte une vérification d'au moins une partie des attributs (ATT-1, ATT-2, ..., ATT-m) médicaux identifiés, comprenant une confirmation et/ou un rejet d'un ou de plusieurs des attributs (ATT-1, ATT-2, ..., ATT-m) identifiés ;
production (S70) d'une information (MEDI) médicale pour le patient reposant sur les constatations (MD2, MD2, MD3) médicales associées aux ensembles (RDS-1, RDs-2, ..., RDS-n) de données de référence, ainsi que sur l'entrée (NE) d'utilisateur comprenant une détermination d'une succession de rang d'au moins une partie des constatations (MD1, MD2, MD3) médicales déjà connues associées aux ensembles (RDS-1, RDs-2, ..., RDS-n) de données de référence reposant sur les similarités (AE-1, AE-2, ..., AE-n) déterminées pour les ensembles (RDS-1, RDs-2, ..., RDS-n) de données de référence respectifs et reposant sur l'entrée (NE) d'utilisateur ; et
mise (S80) à disposition de l'information (MEDI) médicale.

2. Procédé suivant la revendication 1, dans lequel
la mise (S80) à disposition de l'information (MEDI) médicale comprend un affichage (S81) de l'information (MEDI) médicale en passant par l'interface (10) d'utilisateur ; dans lequel
l'affichage (S81) de l'information (MEDI) médicale s'effectue en particulier sous une forme (GUI) de représentation, qui est constituée de manière à ce que l'utilisateur puisse percevoir la succession de rang des constatations (MD1, MD2, MD3) médicales.

3. Procédé suivant l'une des revendications précédentes, dans lequel
les attributs (ATT-1, ATT-2, ..., ATT-m) médicaux comprennent une ou plusieurs des indications suivantes :
une indication, en particulier, démographique, concernant un groupe de patients associé à la constatation (MD1, MD2, MD3) clinique ;
une indication concernant un ou plusieurs symptômes associés à la constatation (MD1, MD2, MD3) médicale respective ;
une constatation concernant un ou plusieurs diagnostics différentiels associés à la constatation (MD1, MD2, MD3) médicale respective ;
une indication concernant un ou plusieurs signes cliniques associés à la constatation (MD1, MD2, MD3) médicale respective ; et/ou
une indication concernant une ou plusieurs contre-indications cliniques associées à la constatation (MD1, MD2, MD3) médicale respective.

4. Procédé suivant l'une des revendications précédentes, dans lequel
le stade de l'identification (S40) des uns ou des plusieurs attributs (ATT-1, ATT-2, ..., ATT-m) médicaux comprend :
mise à disposition d'une affectation (S41), qui affecte un ou plusieurs attributs (ATT-1, ATT-2, ..., ATT-m) à au moins une constatation (MD1, MD2, MD3) médicale déjà connue ; et
affectation (S42) d'une ou de plusieurs attributs (ATT-1, ATT-2, ..., ATT-m) médicaux différents à au moins l'une des constatations (MD1, MD2, MD3) médicales associées aux ensembles (RDS-1, RDS-2, ..., RDS-n) de données de référence en utilisant l'affectation.

5. Procédé suivant l'une des revendications précédentes, comprenant en outre le stade :
production (S30) d'une information (I-MEDI) médicale incidente reposant sur les constatations (MD1, MD2, MD3) médicales associées aux ensembles (RDS-1, RDS-2, ..., RDS-n) de données de référence, reposant en particulier sur les similarités (AE-1, AE-2, ..., AE-n) déterminées pour les ensembles (RDS-1, RDS-2, ..., RDS-n) de données de référence respectifs, dans lequel
le stade de la production (S70) de l'information (MEDI) médicale comprend une adaptation de l'information (I-MEDI) médicale incidente sur la base de l'entrée (NE) d'utilisateur.

6. Procédé suivant l'une des revendications précédentes, dans lequel
l'ensemble (PDS) de données médicales et les ensembles (VDS) de données de comparaison comprennent chacun des données d'image médicale, et
une mesure (AE-1, AE-2, ..., AE-n) de similarité repose respectivement sur une similarité entre les données d'image médicale de l'ensemble (PDS) de données médicales et les données d'image médicale d'un ensemble (VDS) de données de comparaison.

7. Procédé suivant l'une des revendications précédentes, dans lequel
la détermination (S20) du un ou des plusieurs ensembles (RDS-1, RDS-2, ..., RDS-n) de référence comprend :
extraction (S21) d'un descripteur de données à partir de l'ensemble (PDS) de données médicales ;
réception (S22) respectivement d'un descripteur de données correspondant à chacun des ensembles (VDS) de données de comparaison ;
détermination (S23), pour chaque ensemble (VDS) de données de comparaison, d'une mesure de similarité, dans lequel une mesure de similarité repose respectivement sur une similarité entre le descripteur de données et un descripteur de données correspondant ; et
détermination (S24) du un ou des plusieurs ensembles (RDS-1, RDS-2, ..., RDS-n) de données de référence sur la base des mesures de similarité déterminées.

8. Procédé suivant l'une des revendications précédentes, dans lequel
la détermination (S20) du un ou des plusieurs ensembles (RDS-1, RDS-2, ..., RDS-n) de données de référence comprend une application d'une fonction (ALG-1) entraînée, laquelle fonction (ALG-1) entraînée est constituée pour déterminer une mesure (AE-1, AE-2, ..., AE-n) de similarité entre des ensembles (PDS, VDS) de données médicales.

9. Procédé suivant la revendication 8, dans lequel
le stade de la mise (S80) à disposition comprend :
mise (S81) de l'information (MEDI) médicale à disposition de la fonction (ALG-1) entraînée ;
comprenant en outre le stade :
adaptation (T40, T50) de la fonction (ALG-1) entraînée sur la base de l'information (MEDI) médicale.

10. Système (1) pour disposer d'une information (MEDI) médicale comprenant :
une interface (10, 30) et une commande (40), la commande (40) étant constituée :
- pour recevoir un ensemble (PDS) de données médicales d'un patient en passant par l'interface (10, 30) ;
- pour déterminer un ou plusieurs ensembles (RDS-1, RDs-2, ..., RDS-n) de données de référence, à partir de plusieurs ensembles (VDS) de données de comparaison sur la base de mesures (AE-1, AE-2, ..., AE-n) de similarité, dans lequel une mesure (AE-1, AE-2, ..., AE-n) de similarité repose sur une similarité entre l'ensemble (PDS) de données médicales et un ensemble (VDS) de données de comparaison, et dans lequel chaque ensemble (VDS) de données de comparaison est associé à au moins une constatation (MD1, MD2, MD3) médicale déjà connue ;
- pour identifier un ou plusieurs attributs (ATT-1, ATT-2, ..., ATT-m) médicaux, lesquels attributs (ATT-1, ATT-2, ..., ATT-m) médicaux comprennent chacun une caractéristique d'une ou de plusieurs constatations (MD1, MD2, MD3) médicales déjà connues associées aux ensembles (RDS-1, RDs-2, ..., RDS-n) de données de référence, et pour déterminer une succession de rang d'attribut reposant sur les mesures (AE-1, AE-2, ..., AE-n) de similarité déterminées pour les ensembles (RDS-1, RDS-2, ..., RDS-n) de données de référence respectifs et/ou reposant sur un effet discriminant des attributs (ATT-1, ATT-2, ..., ATT-m) concernant la pertinence des constatations (MD1, MD2, MD3) médicales pour le patient et/ou reposant sur une ou plusieurs catégories d'attribut différentes ;
- pour afficher les attributs (ATT-1, ATT-2, ..., ATT-m) médicaux identifiés en passant par l'interface (10, 30) sous une forme de représentation, qui est conformée de manière à ce que l'utilisateur puisse percevoir la succession de rang d'attribut ;
- pour recevoir en passant par l'interface (10, 30) une entrée (NE) d'un utilisateur concernant les attributs (ATT-1, ATT-2, ..., ATT-m) médicaux affichés, dans lequel l'entrée (NE) d'utilisateur comporte une vérification d'au moins une partie des attributs (ATT-1, ATT-2, ..., ATT-m) médicaux identifiés, comprenant une confirmation et/ou un rejet d'un ou de plusieurs des attributs (ATT-1, ATT-2, ..., ATT-m) identifiés ;
- pour produire une information (MEDI) médicale pour le patient reposant sur les constatations (MD1, MD2, MD3) médicales associées aux ensembles (RDS-1, RDS-2, ..., RDS-n) de données de référence, ainsi que sur l'entrée (NE) d'utilisateur comprenant une détermination d'une succession de rang d'au moins une partie des constatations (MD1, MD2, MD3) médicales déjà connues associées aux ensembles (RDS-1, RDs-2, ..., RDS-n) de données de référence reposant sur les similarités (AE-1, AE-2, ..., AE-n) déterminées pour les ensembles (RDS-1, RDs-2, ..., RDS-n) de données de référence respectifs et reposant sur l'entrée (NE) d'utilisateur ; et
- pour disposer de l'information (MEDI) médicale en passant par l'interface (10, 30).

11. Programme d'ordinateur, qui comprend un programme et qui peut être chargé directement dans la mémoire d'une unité informatique programmable d'une commande (40), comprenant des moyens de programme pour exécuter un procédé suivant l'une des revendications 1 à 9, lorsque le programme est exécuté dans la commande (40).

12. Support de mémoire, déchiffrable par ordinateur, sur lequel sont mises en mémoire des parties de programme pouvant être déchiffrées et exécutées, afin d'exécuter tous les stades du procédé suivant l'une des revendications 1 à 9, lorsque les parties sont exécutées par la commande (40).
